Europäisches Patentamt
European Patent Office
Office européen des brevets

(11) Veröffentlichungsnummer: **0 473 963 A1**

(12) **EUROPÄISCHE PATENTANMELDUNG**

(21) Anmeldenummer: **91113388.2**

(22) Anmeldetag: **09.08.91**

(51) Int. Cl.5: **C07D 471/04**, C07D 487/04, C07D 498/04, A61K 31/47, A61K 31/415, //(C07D471/04, 235:00,221:00),(C07D487/04, 235:00,209:00),(C07D498/04, 265:00,235:00)

(30) Priorität: **31.08.90 DE 4027592**

(43) Veröffentlichungstag der Anmeldung: **11.03.92 Patentblatt 92/11**

(84) Benannte Vertragsstaaten: **AT BE CH DE DK ES FR GB IT LI NL SE**

(71) Anmelder: **Beiersdorf Aktiengesellschaft**
**Unnastrasse 48**
**W-2000 Hamburg 20(DE)**

(72) Erfinder: **Paal, Michael, Dr.**
**Hummelsbütteler Kirchenweg 11**
**W-2000 Hamburg 63(DE)**
Erfinder: **Stenzel, Wolfgang, Dr.**
**Lerchenweg 8**
**W-2057 Reinbek(DE)**
Erfinder: **Brückner, Reinhard, Dr.**
**Homburgweg 8**
**W-3000 Hannover 71(DE)**
Erfinder: **Armah, Ben, Dr.**
**Hemmingstedterweg 123f**
**W-2000 Hamburg 52(DE)**

(54) **Neue Pyrrolobenzimidazole, Imidazobenzoxazinone und Imidazochinolone, Verfahren zu ihrer Herstellung und ihre Verwendung sowie die Verbindungen enthaltende Zubereitungen.**

(57) Verbindungen der Formel I

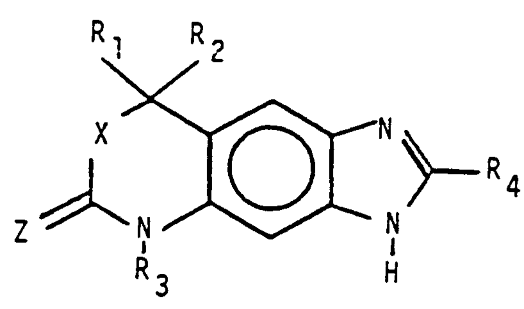

(I)

in der
X die Bedeutung $X_a$, $X_a$l, $X_b$ oder $X_c$ haben kann, und in der im Fall
a), in dem X die Bedeutung $X_a$ hat,
$X_a$ die Methylengruppe $-CH_2-$ bedeutet, und
$R_1$ die Bedeutung $R_{1a}$ hat und
$R_{1a}$ Wasserstoff, $C_{1-6}$-Alkyl, $C_{2-6}$-Alkenyl oder $C_{3-7}$-Cycloalkyl ist und
$R_2$ die Bedeutung $R_{2a}$ hat und
$R_{2a}$ Wasserstoff, $C_{1-6}$-Alkyl, $C_{2-6}$-Alkenyl oder $C_{1-6}$-Hydroxyalkyl bedeutet, wobei $R_{1a}$ und $R_{2a}$ gleich oder verschieden sein können, oder

EP 0 473 963 A1

$R_{1a}$ und $R_{2a}$ zusammen mit dem von ihnen eingeschlossenen Kohlenstoffatom $C_{3-7}$-Spiroalkyl bedeuten,
$R^3$ Wasserstoff oder $C_{1-6}$-Alkyl bedeutet,
$R_4$ die Bedeutung $R_{4a}$ hat und
$R_{4a}$ die Cyanaminogruppe, die 4-Difluormethoxy-3-pyridylgruppe oder eine $C_{1-2}$-Nitroalkylgruppe ist und
Z Sauerstoff oder Schwefel bedeutet,
und in der im Fall
a[1]), in dem X die Bedeutung $X_aI$ hat,
$X_aI$ zusammen mit $R_1$, das in diesem Fall die Bedeutung
$R_{1a}I$ hat, die Gruppe -CH= bedeutet und
$R_2$ die Bedeutung $R_{2a}I$ hat und
$R_{2a}I$ Wasserstoff oder $C_{1-6}$-Alkyl ist,
$R_3$ Wasserstoff oder $C_{1-6}$-Alkyl bedeutet,
$R_4$ die Bedeutung $R_{4a}I$ hat und
$R_{4a}I$ die Cyanaminogruppe, die 4-Difluormethoxy-3-pyridylgruppe, eine $C_{1-2}$-Nitroalkylgruppe, Wasserstoff, die Hydroxygruppe, eine Mercapto-, $C_{1-6}$-Alkylmercapto-, eine Amino-, Pyridylcarbonylamino-, $C_{1-6}$-Alkylcarbonylamino-, eine $C_{1-6}$-Alkyl-, $C_{3-7}$-Cycloalkyl-, $C_{2-6}$-Alkenyl-, $C_{3-7}$-Cycloalkenyl-, $C_{2-6}$-Alkinyl-, $C_{1-6}$-Halogenalkylgruppe oder einen Phenylring der allgemeinen Formel II

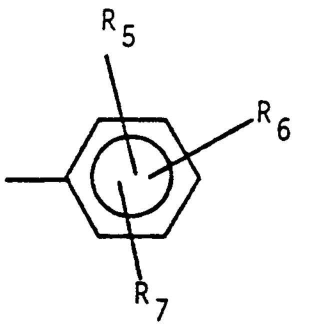

(II)

bedeutet, wobei $R_5$, $R_6$, $R_7$ gleich oder verschieden sein können und jeweils Wasserstoff, eine $C_{1-6}$-Alkansulfonyloxy-, Trifluormethansulfonyloxy-, $C_{1-6}$-Alkansulfonylamino-, Trifluormethansulfonylamino-, N-$C_{1-6}$-Alkyl-$C_{1-6}$-alkylsulfonylamino-, N-$C_{1-6}$-Alkyltrifluormethansulfonylamino-, $C_{1-6}$-Alkylsulfenylmethyl-, $C_{1-6}$-Alkylsulfinylmethyl- oder $C_{1-6}$-Alkylsulfonylmethylgruppe, eine durch eine Hydroxy-, $C_{1-6}$-Alkoxy-, Amino-, $C_{1-6}$-Alkylamino- oder $C_{2-12}$-Dialkylaminogruppe substituierte Carbonylgruppe, eine durch eine Amino-, $C_{1-6}$-Alkylamino-, $C_{2-12}$-Dialkylamino- oder cyclische Iminogruppe substituierte Sulfonylgruppe, wobei eine Methylengruppe in 4-Stellung durch ein Schwefel- oder Sauerstoffatom ersetzt sein kann, eine $C_{1-6}$-Alkylcarbonylamino-, Aminocarbonylamino- oder $C_{1-6}$-Alkylaminocarbonylaminogruppe, eine $C_{1-6}$-Alkylmercapto-, $C_{1-6}$-Alkylsulfinyl- oder $C_{1-6}$-Alkylsulfonylgruppe, eine Nitro-, Halogen-, Amino-, Hydroxy-, $C_{1-6}$-Alkyl-, $C_{1-6}$-Alkoxy-, $C_{1-6}$-Alkenyloxy-, $C_{1-6}$-Alkinyloxy-, Cyan-$C_{1-6}$-alkyloxy-, Carboxy-$C_{1-6}$-alkoxy, $C_{1-6}$-Alkoxycarbonyl-$C_{1-6}$-alkyloxy-, $C_{2-12}$-Dialkylamino-, 1-Imidazolyl-, Trifluormethyl- oder Cyangruppe sein können,
oder $R_{4a}I$ einen Naphthylrest bedeutet
oder $R_{4a}I$ einen gesättigten oder ungesättigten heterocyclischen Fünfring mit 1-4 Heteroatomen oder einen gesättigten oder ungesättigten heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere $C_{1-6}$-Alkyl-, $C_{1-6}$-Alkoxy-, $C_{1-6}$-Alkylmercapto-, Hydroxy-, Nitro-, Amino-, Halogen- oder Cyangruppen substituiert oder mit einem Phenylring zu einem Bicyclus kondensiert sein können und
Z Sauerstoff oder Schwefel bedeutet,
und in der im Fall
b), in dem X die Bedeutung $X_b$ hat,
$X_b$ Sauerstoff bedeutet und
$R_1$ die Bedeutung $R_{1b}$ und $R_2$ die Bedeutung $R_{2b}$ hat und
$R_{1b}$ und $R_{2b}$, die gleich oder verschieden sein können, jeweils für Wasserstoff oder $C_{1-6}$-Alkyl stehen,
$R_3$ Wasserstoff oder $C_{1-6}$-Alkyl bedeutet,
$R_4$ die Bedeutung $R_{4b}$ hat und

$R_{4b}$ die Cyanaminogruppe, die 4-Difluormethoxy-3-pyridylgruppe oder eine $C_{1-2}$-Nitroalkylgruppe ist und
Z Sauerstoff oder Schwefel bedeutet,
und in der im Fall
c), in dem X die Bedeutung $X_c$ hat, $X_c$ eine Bindung bedeutet und
$R_1$ die Bedeutung $R_{1c}$ hat und
$R_{1c}$ Wasserstoff, $C_{1-6}$-Alkyl, $C_{2-6}$-Alkenyl oder $C_{3-7}$-Cycloalkyl ist und
$R_2$ die Bedeutung $R_{2c}$ hat und
$R_{2c}$ Wasserstoff, $C_{1-6}$-Alkyl, $C_{2-6}$-Alkenyl oder $C_{1-6}$-Hydroxyalkyl bedeutet, wobei $R_{1c}$ und $R_{2c}$ gleich oder verschieden sein können, oder
$R_{1c}$ und $R_{2c}$ zusammen mit dem von ihnen eingeschlossenen Kohlenstoffatom $C_{3-7}$-Spiroalkyl bedeuten,
$R_3$ Wasserstoff oder $C_{1-6}$-Alkyl bedeutet,
$R_4$ die Bedeutung $R_{4c}$ hat und
$R_{4c}$ die Cyanaminogruppe, die 4-Difluormethoxy-3-pyridylgruppe oder eine $C_{1-2}$-Nitroalkylgruppe ist und
Z Sauerstoff oder Schwefel bedeutet,
sowie deren Salze und Säureadditionssalze, Tautomere und optische Isomere sind PDE-III- und PDE-IV-Hemmstoffe.

Die Erfindung betrifft neue Verbindungen der Formel I

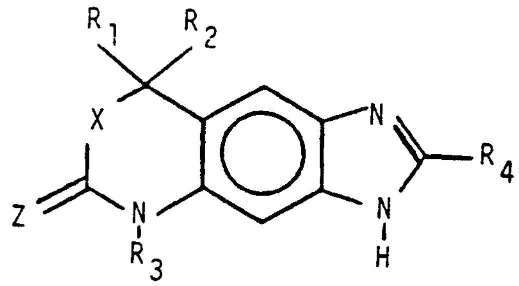

(I)

in der
X die Bedeutung $X_a$, $X_al$, $X_b$ oder $X_c$ haben kann, und in der im Fall
a), in dem X die Bedeutung $X_a$ hat,
$X_a$ die Methylengruppe $-CH_2-$ bedeutet, und
$R_1$ die Bedeutung $R_{1a}$ hat und
$R_{1a}$ Wasserstoff, $C_{1-6}$-Alkyl, $C_{2-6}$-Alkenyl oder $C_{3-7}$-Cycloalkyl ist und
$R_2$ die Bedeutung $R_{2a}$ hat und
$R_{2a}$ Wasserstoff, $C_{1-6}$-Alkyl, $C_{2-6}$-Alkenyl oder $C_{1-6}$-Hydroxyalkyl bedeutet, wobei $R_{1a}$ und $R_{2a}$ gleich oder verschieden sein können, oder
$R_{1a}$ und $R_{2a}$ zusammen mit dem von ihnen eingeschlossenen Kohlenstoffatom $C_{3-7}$-Spiroalkyl bedeuten,
$R^3$ Wasserstoff oder $C_{1-6}$-Alkyl bedeutet,
$R_4$ die Bedeutung $R_{4a}$ hat und
$R_{4a}$ die Cyanaminogruppe, die 4-Difluormethoxy-3-pyridylgruppe oder eine $C_{1-2}$-Nitroalkylgruppe ist und
Z Sauerstoff oder Schwefel bedeutet,
und in der im Fall
$a^1$), in dem X die Bedeutung $X_al$ hat,
$X_al$ zusammen mit $R_1$, das in diesem Fall die Bedeutung
$R_{1a}l$ hat, die Gruppe -CH= bedeutet und
$R_2$ die Bedeutung $R_{2a}l$ hat und
$R_{2a}l$ Wasserstoff oder $C_{1-6}$-Alkyl ist,
$R_3$ Wasserstoff oder $C_{1-6}$-Alkyl bedeutet,
$R_4$ die Bedeutung $R_{4a}l$ hat und
$R_{4a}l$ die Cyanaminogruppe, die 4-Difluormethoxy-3-pyridylgruppe, eine $C_{1-2}$-Nitroalkylgruppe, Wasserstoff, die Hydroxygruppe, eine Mercapto-, $C_{1-6}$-Alkylmercapto-, eine Amino-, Pyridylcarbonylamino-, $C_{1-6}$-Alkylcarbonylamino-, eine $C_{1-6}$-Alkyl-, $C_{3-7}$-Cycloalkyl-, $C_{2-6}$-Alkenyl-, $C_{3-7}$-Cycloalkenyl-, $C_{2-6}$-Alkinyl-, $C_{1-6}$-Halogenalkylgruppe oder einen Phenylring der allgemeinen Formel II

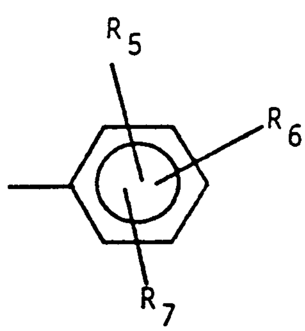

(II)

bedeutet, wobei $R_5$, $R_6$, $R_7$ gleich oder verschieden sein können und jeweils Wasserstoff, eine $C_{1-6}$-Alkansulfonyloxy-, Trifluormethansulfonyloxy-, $C_{1-6}$-Alkansulfonylamino-, Trifluormethansulfonylamino-, N-$C_{1-6}$-Alkyl-$C_{1-6}$-alkylsulfonylamino-, N-$C_{1-6}$-Alkyltrifluormethansulfonylamino-, $C_{1-6}$-Alkylsulfenylmethyl-, $C_{1-6}$-Alkylsulfinylmethyl- oder $C_{1-6}$-Alkylsulfonylmethylgruppe, eine durch eine Hydroxy-, $C_{1-6}$-Alkoxy-, Amino-, $C_{1-6}$-Alkylamino- oder $C_{2-12}$-Dialkylaminogruppe substituierte Carbonylgruppe, eine durch eine Amino-, $C_{1-6}$-Alkylamino-, $C_{2-12}$-Dialkylamino- oder cyclische Iminogruppe substituierte Sulfonylgruppe, wobei eine Methylengruppe in 4-Stellung durch ein Schwefel- oder Sauerstoffatom ersetzt sein kann, eine $C_{1-6}$-Alkylcarbonylamino-, Aminocarbonylamino- oder $C_{1-6}$-Alkylamino-

carbonylaminogruppe, eine $C_{1-6}$-Alkylmercapto-, $C_{1-6}$-Alkylsulfinyl- oder $C_{1-6}$-Alkylsulfonylgruppe, eine Nitro-, Halogen-, Amino-, Hydroxy-, $C_{1-6}$-Alkyl-, $C_{1-6}$-Alkoxy-,$C_{1-6}$-Alkenyloxy-, $C_{1-6}$-Alkinyloxy-, Cyan-$C_{1-6}$-alkyloxy-, Carboxy-$C_{1-6}$-alkoxy, $C_{1-6}$-Alkoxycarbonyl-$C_{1-6}$-alkyloxy-, $C_{2-12}$-Dialkylamino-, 1-Imidazolyl-, Trifluormethyl- oder Cyangruppe sein können,
oder $R_{4a}$l einen Naphthylrest bedeutet
oder $R_{4a}$l einen gesättigten oder ungesättigten heterocyclischen Fünfring mit 1-4 Heteroatomen oder einen gesättigten oder ungesättigten heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere $C_{1-6}$-Alkyl-, $C_{1-6}$-Alkoxy-, $C_{1-6}$-Alkylmercapto-, Hydroxy-, Nitro-, Amino-, Halogen- oder Cyangruppen substituiert oder mit einem Phenylring zu einem Bicyclus kondensiert sein können und
Z Sauerstoff oder Schwefel bedeutet,
und in der im Fall
b), in dem X die Bedeutung $X_b$ hat,
$X_b$ Sauerstoff bedeutet und
$R_1$ die Bedeutung $R_{1b}$ und $R_2$ die Bedeutung $R_{2b}$ hat und
$R_{1b}$ und $R_{2b}$, die gleich oder verschieden sein können,
jeweils für Wasserstoff oder $C_{1-6}$-Alkyl stehen,
$R_3$ Wasserstoff oder $C_{1-6}$-Alkyl bedeutet,
$R_4$ die Bedeutung $R_{4b}$ hat und
$R_{4b}$ die Cyanaminogruppe, die 4-Difluormethoxy-3-pyridylgruppe oder eine $C_{1-2}$-Nitroalkylgruppe ist und
Z Sauerstoff oder Schwefel bedeutet,
und in der im Fall
c), in dem X die Bedeutung $X_c$ hat, $X_c$ eine Bindung bedeutet und
$R_1$ die Bedeutung $R_{1c}$ hat und
$R_{1c}$ Wasserstoff, $C_{1-6}$-Alkyl, $C_{2-6}$-Alkenyl oder $C_{3-7}$-Cycloalkyl ist und
$R_2$ die Bedeutung $R_{2c}$ hat und
$R_{2c}$ Wasserstoff, $C_{1-6}$-Alkyl, $C_{2-6}$-Alkenyl oder $C_{1-6}$-Hydroxyalkyl bedeutet, wobei $R_{1c}$ und $R_{2c}$ gleich oder verschieden sein können, oder
$R_{1c}$ und $R_{2c}$ zusammen mit dem von ihnen eingeschlossenen Kohlenstoffatom $C_{3-7}$-Spiroalkyl bedeuten,
$R_3$ Wasserstoff oder $C_{1-6}$-Alkyl bedeutet,
$R_4$ die Bedeutung $R_{4c}$ hat und
$R_{4c}$ die Cyanaminogruppe, die 4-Difluormethoxy-3-pyridylgruppe oder eine $C_{1-2}$-Nitroalkylgruppe ist und
Z Sauerstoff oder Schwefel bedeutet,
sowie deren Salze und Säureadditionssalze, Tautomere und optische Isomere, Verfahren zu ihrer Herstellung, ihre Verwendung und Zubereitungen, die diese Verbindungen enthalten.

Der Einfachheit halber sind die erfindungsgemäßen Verbindungen in nur einer durch Formel I wiedergegebenen tautomeren Form definiert. Die Erfindung erstreckt sich jedoch auf alle tautomeren Formen der Verbindungen.

Obgleich pharmazeutisch verträgliche Salze und Säureadditionssalze der neuen Verbindungen der Formel I und deren tautomere Formen bevorzugt sind, liegen alle Salze innerhalb des Bereichs der Erfindung. Alle Salze sind wertvoll zur Herstellung der Verbindungen, selbst wenn das spezielle Salz nur als Zwischenprodukt gewünscht wird, wie zum Beispiel, wenn das Salz nur für Zwecke der Reinigung oder Identifizierung gebildet wird, oder wenn es als ein Zwischenprodukt bei der Herstellung eines pharmazeutisch verträglichen Salzes, beispielsweise durch Ionenaustauschverfahrensweise, verwendet wird.

Die Verbindungen der allgemeinen Formel I und deren Salze können asymmetrische Kohlenstoffatome enthalten. Daher sind auch die verschiedenen optischen Isomeren sowie die Diastereoisomeren Gegenstand der Erfindung ebenso wie die Salze und Additionssalze dieser Verbindungen mit Säuren. Die Racemate können nach an sich bekannten Methoden in ihre optischen Antipoden aufgetrennt werden.

Verbindungen des Falles a) mit $X = X_a$ und den zugehörigen vorstehend genannten Substituenten sind 7,8-Dihydro-imidazochinolone und entsprechend die des Falles $a^1$) mit $X = X_a$l sind Imidazochinolone bzw. mit Z = S jeweils deren Thioanaloge.

Die Verbindungen des Falles b) mit $X = X_b$ und den zugehörigen vorstehend genannten Substituenten bilden Imidazobenzoxazinone bzw. mit Z = S jeweils deren Thioanaloge.

In den deutschen Offenlegungsschriften 3 417 643, 3 501 497, 3 524 067, 3 531 678, 3 639 466 und 3 642 315 werden in 2-Stellung unterschiedlich substituierte Pyrrolobenzimidazole, in der DE-A-3 633 861 Imidazobenzoxazinone und der DE-A-3 701 277 Imidazochinolinone und diese Verbindungen enthaltende

Arzneimittel zur Behandlung von Herz- und Kreislauferkrankungen beschrieben.

Die vorliegende Erfindung betrifft dagegen in 2-Stellung neuartig substituierte Pyrrolobenzimidazole (X = Valenz), Imidazobenzoxazinone (X = O) und Imidazochinolone (X = $-CH_2-$, -CH=). Sie werden weder spezifisch offenbart noch nahegelegt.

Mit den folgenden Formeln Ia, Ia[1], Ib und Ic sind die zu den vorstehend genannten Fällen a), a[1]), b) und c) gehörenden Teilformeln für die entsprechenden genannten Bedeutungen von X in diesen Formeln wiedergegeben, wobei hier also $X_a$: $CH_2$, $X_al$ : -CH, $X_b$ : O und $X_c$ : eine Bindung ist:

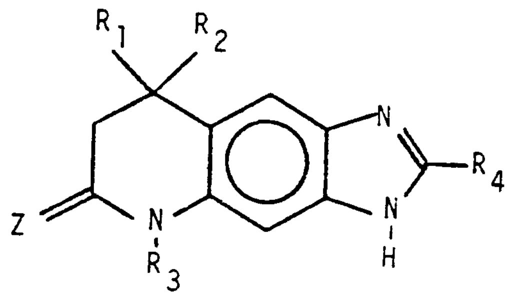

(Ia)

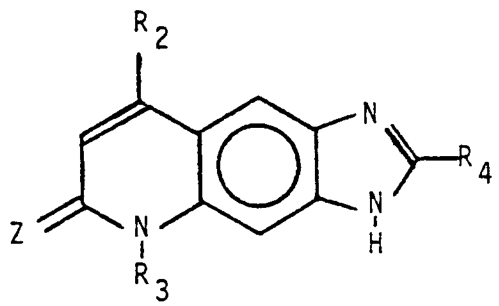

$(Ia^1)$

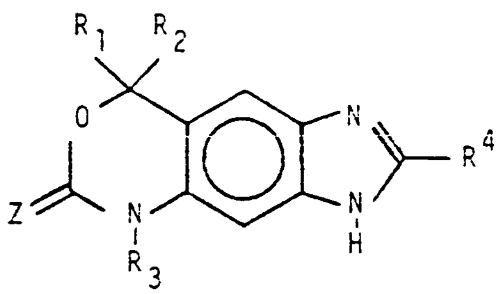

(Ib)

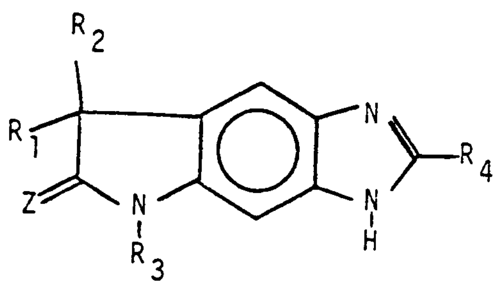

(Ic)

und die übrigen Substituenten die jeweils vorstehend angegebene Bedeutung haben.

Besonders bevorzugt werden die Pyrrolobenzimidazole der Formel $I_c$ entsprechend dem vorstehend beschriebenen Fall c), in dem X bzw. $X_c$ eine Bindung bedeutet,

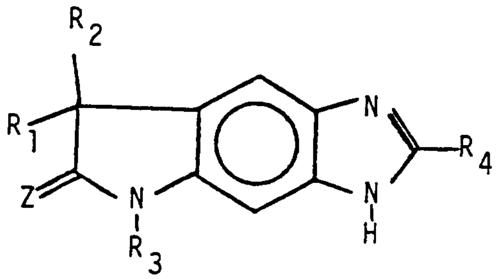

(Ic)

in der $R_{1c}$, $R_{2c}$, $R_3$, $R_{4c}$ und Z die vorstehend angegebene Bedeutung haben, und deren Salze und Säureadditionssalze.

Die erfindungsgemäßen Alkylgruppen und Alkylteile beziehungsweise Alkylenteile von Gruppen können geradkettig oder verzweigt sein und sie besitzen, soweit nicht anders angegeben, jeweils bevorzugt 1 bis 6 Kohlenstoffatome, vorzugsweise 1 bis 4 Kohlenstoffatome, insbesondere 1 oder 2 Kohlenstoffatome. Die verzweigten Alkylgruppen besitzen mindestens 3 Kohlenstoffatome. Bevorzugte Alkyl- oder Alkylenteile sind Methyl, Ethyl, n-Propyl, Isopropyl, Butyl beziehungsweise entsprechend Methylen, Ethylen, n- oder Isopropylen und Butylen.

Die erfindungsgemäßen Alken- und Alkingruppen und Alken- oder Alkinteile von Gruppen können geradkettig oder verzweigt sein und sie besitzen, soweit nicht anders angegeben, jeweils bevorzugt 2 bis 6 Kohlenstoffatome, vorzugsweise 2 bis 4 Kohlenstoffatome, insbesondere 2 oder 3 Kohlenstoffatome.

Vorzugsweise besitzen erfindungsgemäße Cycloalkylgruppen und Cycloalkylteile wie Cycloalkylreste von Cycloalkylalkylgruppen 3 - 7 Kohlenstoffatome, insbesondere 3 bis 6 Kohlenstoffatome. Besonders bevorzugt sind Cyclopropyl und Cyclohexyl.

Vorzugsweise besitzen erfindungsgemäße Cycloalkylengruppen und Cycloalkylenteile von Gruppen 3 bis 6 Kohlenstoffatome.

Halogen ist Fluor, Chlor, Brom und Jod. Bevorzugt wird Fluor, Chlor oder Brom.

Pyridylcarbonylamino ist 2-, 3- oder 4-Pyridinyl-CO-NH-, $C_{1-6}$-Alkylcarbonylamino ist $C_{1-6}$-Alkyl-CO-NH-, $C_{1-6}$-Alkylaminocarbonylamino ist $C_{1-6}$-Alkyl-NH-CO-NH, $C_{1-6}$-Alkoxycarbonyl ist $C_{1-6}$-Alkoxy-CO-.

$C_{1-2}$-Nitroalkyl ist vorzugsweise $O_2N$-$CH_2$-.Halogenalkylgruppen besitzen vorzugsweise 1-3 Halogenatome, insbesondere ein oder zwei Halogenatome.

Vorzugsweise ist $R_4$ die Cyanaminogruppe -NH-CN.

Bedeutet $R_{4a}$l einen Phenylring der allgemeinen Formel II so kann der Alkylteil der bei $R_5$, $R_6$, $R_7$ genannten Substituenten auch 1 - 5 Kohlenstoffatome enthalten, vorzugsweise 1 - 4 Kohlenstoffatome. Bevorzugt in diesem Sinne sind beispielsweise die Methansulfonyloxy-, Ethansulfonyloxy-, n-Propansulfonyloxy-, Isopropansulfonyloxy-, Trifluormethansulfonyloxy-, Methylsulfenylmethyl-, Ethylsulfenylmethyl-, n-Propylsulfenylmethyl-, Methylsulfinylmethyl-, Ethylsulfinylmethyl-, Methylsulfonylmethyl-, Ethylsulfonylmethyl-, n-Propylsulfonylmethyl-, Methansulfonylamino-, Ethansulfonylamino-, n-Propansulfonylamino-, Trifluormethansulfonylamino-, N-Methyl-methansulfonylamino-, N-Ethyl-methansulfonylamino-, N-Methyl-ethansulfonylamino-, N-Ethyl-ethansulfonylamino-, N-Isopropylethansulfonylamino-, N-Methyl-n-propansulfonylamino-, N-n-Propyl-n-propansulfonylamino-, N-Methyltrifluormethansulfonylamino-, N-Ethyl-trifluormethansulfonylamino-, N-Isopropyl-trifluormethansulfonylamino-, Methoxycarbonyl-, Ethoxycarbonyl-, Propoxycarbonyl-, Isopropoxycarbonyl-, Methylaminocarbonyl-, Ethylaminocarbonyl-, Dimethylaminocarbonyl-, Di-n-propylaminocarbonyl-, N-Methyl-ethylaminocarbonyl-, Trifluormethyl-, Methylaminosulfonyl-, Ethylaminosulfonyl-, n-Propylaminosulfonyl, n-Butylaminosulfonyl-, n-Pentylaminosulfonyl-, Dimethylaminosulfonyl-, Diethylaminosulfonyl-, Di-n-propylaminosulfonyl-, N-Methylisopropylaminosulfonyl-, Acetylamino-, Propionylamino-, Methylcarbonylamino-, Ethylaminocarbonylamino- oder Propylaminocarbonylaminogruppe, eine Methyl-, Ethyl-, Propyl-, Methoxy-, Ethoxy-, Propyloxy-, Allyloxy-, 2-Butenyloxy-, 3-Butenyloxy-, 2-Pentenyloxy-, Propargyloxy-, 2-Butinyloxy-, 3-Butinyloxy-, Cyanmethyloxy-, Cyanethyloxy-, Methoxycarbonylmethyloxy-, Methoxycarbonylethyloxy-, Methylmercapto-, Ethylmercapto-, Methylsulfinyl-, Ethylsulfinyl-, Methylsulfonyl- oder Ethylsulfonylgruppe.

Bei Sulfonylgruppen, die durch cyclische Iminogruppen substituiert sein können, sind bevorzugt die Morpholino-, Pyrrolidino-, Piperidino- und Hexamethyleniminosulfonylgruppen.

Insbesondere sind bevorzugt für $R_5$ Wasserstoff, eine Alkylsulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino oder Dialkylaminogruppe substituierte Carbonylgruppe oder eine durch eine Amino-, Dialkylamino- oder Morpholinogruppe substituierte Sulfonylgruppe, wobei jeder der

vorstehend genannten Alkylteile 1 oder 2 Kohlenstoffatome enthalten kann, eine Nitro-, Cyan- oder Alkylaminosulfonylgruppe mit 1 - 4 Kohlenstoffatomen, eine Alkylcarbonylamino-, Aminocarbonylamino- oder N-Alkyl-aminocarbonylaminogruppe, eine Alkylmercapto-, Alkylsulfinyl- oder Alkylsulfonylgruppe, wobei jeder der vorgenannten Alkylteile 1 oder 2 Kohlenstoffatome enthalten kann, eine Halogen-, Amino-, Hydroxy-, Dialkylamino-, Alkyl-, Alkoxy-, Alkenyloxy- oder Alkinyloxygruppe vorzugsweise mit 1 - 3 Kohlenstoffatomen, eine Cyanmethyloxy- oder Methoxycarbonylmethyloxylgruppe, die Trifluormethylgruppe oder die 1-Imidazoylgruppe, für $R_6$ Wasserstoff, eine Alkylgruppe mit 1 - 3 Kohlenstoffatomen, eine Alkoxy- oder Dialkylaminogruppe mit 1 bis 2 Kohlenstoffatomen an jedem Alkylteil oder ein Halogenatom und für $R_7$ Wasserstoff oder die Methoxygruppe.

Der Phenylteil kann 1 bis 3 der genannten Substituenten tragen.

Bevorzugte monosubstituierte Phenylverbindungen sind die Hydroxy-, $C_1$ - $C_3$-Alkyl-, $C_1$ - $C_3$-Alkoxy-, Allyloxy-, Propargyloxy-, Cyanmethyloxy-, Methoxycarbonylmethyloxy-, Halogen-, Nitro-, Cyan-, Aminocarbonyl-, Methoxycarbonyl-, Amino-, $C_1$ - $C_3$-Dialkylamino-, $C_1$ - $C_3$-Alkylmercapto-, $C_1$ - $C_3$-Alkylsulfinyl-, $C_1$ - $C_3$-Alkylsulfonyl-, $C_1$ - $C_3$-Alkylsulfonyloxy- und die 1-Imidazolyl-phenyle, wobei der Substituent in 2-, 3- oder 4-Stellung stehen kann.

Bevorzugte disubstituierte Phenyle enthalten als Substituenten eine Alkansulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, eine Alkylaminosulfonyl-, Alkylcarbonylamino-, Aminocarbonylamino- oder N-Alkyl-aminocarbonylaminogruppe, eine Hydroxy-, Alkyl-, Alkoxy-, Allyloxy-, Propargyloxy-, Cyanmethyloxy-, Methoxycarbonylmethyloxy-, Cyan-, Halogen-, Nitro-, Amino-, Dialkylamino-, Alkylmercapto-, Alkylsulfinyl-, Alkylsulfonyl- oder eine 1-Imidazolylgruppe, wobei die beiden Substituenten gleich oder verschieden sein können und in 2,3-, 2,4-, 2,5-, 2,6-, 3,4- und 3,5-Stellung bevorzugt jedoch in 2,4-, 2,5- und 3,4-Stellung stehen können und die vorgenannten Alkylreste, allein oder in Kombination mit anderen Resten, 1 - 3 C-Atome aufweisen können.

Bevorzugter trisubstituierter Phenylrest ist der 3,4,5-Trimethoxyphenylrest.

Bedeutet $R_{4a}$l einen heterocyclischen Fünfring mit 1 - 4 Heteroatomen oder einen heterocyclischen Sechsring mit 1 - 5 Heteroatomen, wobei die Heteroatome der vorgenannten Fünf- oder Sechsringe gleich oder verschieden sein können und Stickstoff, Sauerstoff oder Schwefel bedeuten und gegebenenfalls an einem oder mehreren Stickstoffatomen ein Sauerstoff tragen können, so sind in diesem Sinne bevorzugt der Pyrrol-, Furan-, Thiophen-, Pyrazol-, Imidazol-, Thiazol-, Isothiazol-, Oxazol-, Isoxazol-, Triazol-, Tetrazol-, Thiadiazol-, Oxadiazol-, Pyrazin-, N,N'-Dioxy-pyrazin-, Pyrimidin-, N,N'-Dioxy-pyrimidin-, Pyridazin-, Oxazin-, Thiazin-, Triazin-, Tetrazin-, Pyridin-, N-Oxy-pyridin-, Piperidin-, Piperazin-, Morpholin- und Thiomorpholinrest.

Alkyl-, Alkoxy- und Alkylmercapto-Substituenten in den heterocyclischen Fünf- und Sechsringen können 1 - 6, vorzugsweise 1 - 4 Kohlenstoffatome enthalten. Bevorzugt ist der Methyl-, Ethyl-, Methoxy-, Ethoxy-, Methylmercapto- und Ethylmercaptorest. Unter Halogen ist Fluor, Chlor und Brom, vorzugsweise Chlor zu verstehen.

Sind die heterocyclischen Fünf- und Sechsringe mit einem Phenylring kondensiert, so sind bevorzugt der Indol-, Indazol-, Benzimidazol-, Chinolin-, Isochinolin-, Cinnolin-, Phthalazin-, Chinazolin-, Chinoxalin-, Benzofuran-, Benzothiophen-, Benzoxazol-, Benzisoxazol-, Benzothiazol- und der Benzisothiazolrest, außerdem der Naphthylrest.

Bedeutet $R_{4a}$l einen Alkyl-, Alkenyl- oder Alkinylrest, so sind darunter gerade oder verzweigte Ketten mit bis zu sechs C-Atomen zu verstehen. Bevorzugt in diesem Sinne ist der Methyl-, Ethyl-, Propyl-, Butyl-, Pentyl-, Hexyl-, Vinyl-, Propenyl- und Propinylrest. Bedeutet $R_{4a}$l einen Cycloalkyl- oder Cycloalkenylrest, so sind darunter Ringe mit drei bis sieben Gliedern zu verstehen. Bevorzugt in diesem Sinne ist der Cyclopropyl-, Cyclobutyl-, Cyclopentyl-, Cyclohexyl-, Cyclopentenyl- und Cyclohexenylrest. Bedeutet $R_{4a}$l einen Halogenalkyl-, Alkoxyalkyl-, Carboxyalkyl-, Alkoxycarbonylalkyl-, Hydroxyalkyl-, Aminoalkyl-, Alkoxycarbonylaminoalkyl-, Alkylsulfonylaminoalkyl-, Alkylmercapto- oder einen Alkylcarbonylaminorest, so können die Alkyl- oder Alkoxygruppen vorzugsweise 1 bis 4 C-Atome enthalten. Unter Halogen ist Fluor und Chlor, vorzugsweise Fluor zu verstehen.

Bevorzugt in diesem Sinne ist der Trifluormethyl-, Ethoxymethyl-, Methoxyethyl-, Ethoxyethyl-, Carboxymethyl-, Carboxypropyl-, Carboxybutyl-, Methoxycarbonylmethyl-, Methoxycarbonylethyl-, Methoxycarbonylpropyl-, Ethoxycarbonylmethyl-, Ethoxycarbonylethyl-, Ethoxycarbonylpropyl-, Propoxycarbonylethyl-, Aminomethyl-, Aminoethyl-, Aminopropyl-, Aminobutyl-, Hydroxymethyl-, Hydroxyethyl-, Hydroxypropyl-, Hydroxybutyl-, Methylmercapto-, Ethylmercapto-, Propylmercapto-,

Butylmercapto-, Acetylamino-, Propionylamino-, Butyloxycarbonylamino-, Methylsulfonylamino-, Formylaminopropyl-, Acetylaminopropyl-, Propionylaminopropyl- und der Methylsulfonylaminopropylrest.

Bedeuten in der allgemeinen Formel I $R_1$ und $R_2$ Alkylgruppen, so sind darunter gerade oder verzweigte Alkylketten mit 1 - 6 C-Atomen zu verstehen. Bevorzugt in diesem Sinne sind die Methyl-, Ethyl-, Propyl- und die Butylgruppe.

Bilden $R_1$ und $R_2$ zusammen mit dem C-Atom, an das sie gebunden sind einen carbocyclischen Ring, so sind darunter Ringe mit drei bis sieben Gliedern zu verstehen. Bevorzugt sind der Cyclopropan-, Cyclobutan-, Cyclopentan- und der Cyclohexanring.

Besonders bevorzugte Verbindungen sind Verbindungen der allgemeinen Formel I in der $R_4$ die Cyanaminogruppe oder $R_4$ den Phenylrest der allgemeinen Formel II bedeutet, in der $R_5$ ein Wasserstoffatom, die Methansulfonyloxy-, Trifluormethansulfonyloxy-, Methansulfonylamino-, Trifluormethansulfonylamino-, Methylmercapto-, Methylsulfinyl-, Methylsulfonyl-, Hydroxy-, Methyl-, Methoxy-, Propargyloxy-, Trifluormethyl- oder die 1-Imidazolylgruppe,
$R_6$ Wasserstoff, die Methyl-, Methoxy-, Dimethylamino- oder Chlorgruppe, $R_7$ Wasserstoff oder die Methoxygruppe bedeutet oder $R_4$ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyridin-, N-Oxy-pyridin-, Pyrazin-, N,N'-Dioxypyrazin, Pyrimidin-, N,N'-Dioxypyrimidin-, Pyridazin-, Oxazin-, Thiazin-, Triazin-, Tetrazin-, Piperidin-, Piperazin-, Morpholin- oder Thiomorpholinrest darstellt sowie deren Methyl-, Ethyl-, Methoxy-, Ethoxy-, Methylmercapto-, Ethylmercapto- und Chlor-substituierte Derivate oder den Indol-, Indazol-, Chinolin- oder Isochinolinrest bedeutet oder einen Naphthylrest darstellt, $R_1$ und $R_2$ gleich sind und Methylgruppen bedeuten oder $R_1$ und $R_2$ zusammen mit dem Kohlenstoffatom, an das sie gebunden sind, einen Cyclopentanring oder Cyclohexanring bilden und $R_3$ Wasserstoff, Methyl, Ethyl oder Propyl bedeutet.

Die folgenden erfindungsgemäßen Verbindungen, deren Salze und Säureadditionssalze, Tautomere und optische Isomere werden bevorzugt:
2-Cyanamino-6,7-dihydro-7,7-dimethyl-3H,5H-pyrrolo(2,3-f)benzimidazol-6-on
2-Cyanamino-5,7,7-trimethyl-6,7-dihydro-3H-pyrrolo(2,3-f)benzimidazol-6-on
2-Cyanamino-6,7-dihydro-7,7-cyclopropyl-3H,5H-pyrrolo(2,3-f)benzimidazol-6-on
2-Nitromethyl-6,7-dihydro-7,7-dimethyl-3H,5H-pyrrolo(2,3-f)benzimidazol-6-on
2-((4-Difluormethoxy)-3-pyridyl)-6,7-dihydro-7,7-dimethyl-3H,5H-pyrrolo(2,3-f)-benzimidazol-6-on
2-Cyanamino-8,8-dimethyl-8-hydro-5H-imidazo(4,5-g)(3,1) benzoxazin-6-on
8,8-Dimethyl-2-nitromethyl-8-hydro-5H-imidazo(4,5-g)(3,1)benzoxazin-6-on
8,8-Dimethyl-2-(4-difluormethoxy-3-pyridyl)-8-hydro-5H-imidazo(4,5-g)(3,1)benzoxazin-6-on
2-Methyl-3H,5H-imidazo(4,5-g)chinolin-6-on
2-Trifluormethyl-3H,5H-imidazo(4,5-g)chinolin-6-on
2-(4-pyridyl)-3H,5H-imidazo(4,5-g)chinolin-6-on
2-(3-pyridyl)-3H,5H-imidazo (4,5-g)chinolin-6-on
2-Cyanamino-7,8-dihydro-3H,5H-imidazo(4,5-g)chinolin-6-on
2-Cyanamino-3H,5H-imidazo(4,5-g)chinolin-6-on
2-(4-Methylthio-phenyl)-3H,5H-imidazo(4,5-g)chinolin-6-on
2-(4-Methoxy-phenyl)-3H,5H-imidazo(4,5-g)chinolin-6-on
2-(4-(1H-Imidazol-1-yl)-phenyl)-3H,5H-imidazo(4,5-g)chinolin-6-on
2-(4-(Trimethylsulfinyl)-phenyl-3H,5H-imidazo-(4,5-g) chinolin-6-on

Die erfindungsgemäßen Verbindungen der Formel I, ihre physiologisch verträglichen Salze und Säureadditionssalze sowie deren tautomere und optische Isomere sind therapeutische Wirkstoffe, besitzen hohe pharmakologische Wirkung und sind wertvolle Arzneimittel. Beispielsweise sind sie PDE-III-Hemmstoffe und PDE-IV-Hemmstoffe. Sie weisen vorzugsweise kardiovaskuläre Eigenschaften und Wirkungen auf. Insbesondere zeichnen sie sich durch vasodilatierende, blutdrucksenkende, positiv inotrope und antithrombotische Wirkungen aus.

Die erfindungsgemäßen Verbindungen sind zur Behandlung und Prophylaxe der chronischen und akuten Herzinsuffizienz unterschiedlicher Genese und/oder zur Behandlung und Prophylaxe arterieller Thromboembolien und arterieller Verschlußkrankheiten bei Warmblütern, insbesondere dem Menschen geeignet.

Die erfindungsgemäßen Verbindungen haben insbesondere blutdrucksenkende Wirkung und können somit als antihypertensive Mittel zur Behandlung und Prophylaxe der Hypertonie verwendet werden.

Außerdem können sie zur Behandlung und Prophylaxe von Psoriasis, Neurodermitis und Asthma, insbesondere Asthma bronchiale, dienen. Die Verbindungen hemmen die Thrombozytenaggregation. Sie können zur Behandlung und Prophylaxe von thromboembolischen Erkrankungen dienen.

Die Verbindungen der vorliegenden Erfindung können oral oder parenteral angewendet werden.

Hierzu lassen sich die neuen Verbindungen, gegebenenfalls in Kombination mit anderen Wirksubstanzen, insbesondere in die üblichen pharmazeutischen Anwendungsformen wie Tabletten, Dragees, Pulver, Suspensionen, Suppositorien oder Ampullen einarbeiten. Beispielsweise beträgt die Einzeldosis hierbei beim Erwachsenen jeweils einmal bis viermal täglich bei intravenöser Applikation 1 - 50 mg, vorzugsweise 2 bis 40 mg, und bei oraler Applikation 5 - 150 mg, vorzugsweise 5 bis 100 mg.

Die erfindungsgemäßen Verbindungen werden üblicherweise in Mengen von 10 - 500 mg pro Tag bezogen auf 75 kg Körpergewicht appliziert. Bevorzugt ist es, 2 - 3mal pro Tag 1-2 Tabletten mit einem Wirkstoffgehalt von 2-20 mg zu verabreichen. Die Tabletten können auch retardiert sein, wodurch nur noch 1mal pro Tag 1-2 Tabletten mit 10-500 mg Wirkstoff gegeben werden muß. Der Wirkstoff kann auch durch Injektion 1 - 8mal pro Tag bzw. durch Dauerinfusion gegeben werden, wobei Mengen von 5 - 200 mg/Tag normalerweise ausreichen. Diese Dosierungen sind vorteilhaft für die Behandlung der genannten Krankheiten, insbesondere der Hypertonie.

Die erfindungsgemäßen Substanzen zeichnen sich durch eine beträchtliche Senkung des arteriellen Blutdrucks aus. Dosen von etwa 1 mg/kg. p.o. führen zu einer Senkung des Blutdrucks um mindestens 20% an hypertensiven Ratten.

Trotzdem kann es gegebenenfalls erforderlich sein, von den genannten Mengen abzuweichen, und zwar in Abhängigkeit vom Körpergewicht beziehungsweise der Art des Applikationsweges, aber auch aufgrund des individuellen Verhaltens gegenüber dem Mekikament beziehungsweise der Art von dessen Formulierung und dem Zeitpunkt beziehungsweise Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen.

Die Behandlung von Hautkrankheiten, Neurodermitis oder Psoriasis kann auch topisch erfolgen. Zur Behandlung sind beispielsweise Cremes, Lotionen, Salben, Lösungen oder Puder geeignet, die den Wirkstoff vorzugsweise in einer Menge von 1 - 10% enthalten. Zur Prophylaxe oder Behandlung wird eine topische Zubereitung auf die Haut oder die erkrankte Haut einmal oder mehrmals täglich in dünner Schicht aufgetragen.

Gegenstand der Erfindung sind auch die erfindungsgemäßen Verbindungen zur Behandlung der vorstehenden Krankheiten sowie Verfahren zur Behandlung dieser Krankheiten, in denen diese Verbindungen verwendet werden sowie ihre Verwendung in Verfahren zur Herstellung von Mitteln, die diese Verbindungen enthalten, zur Behandlung dieser Krankheiten sowie Verfahren zur Herstellung der Verbindungen.

Gemäß der Erfindung werden pharmazeutische Präparate oder Zusammensetzungen geschaffen, die eine erfindungsgemäße Verbindung oder deren pharmazeutisch verträgliches Salz oder Säureadditionssalz gegebenenfalls zusammen mit einem pharmazeutisch verträglichen Verdünnungsmittel oder Träger enthalten sowie topische Zubereitungen mit diesen erfindungsgemäßen Verbindungen.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können vorzugsweise oral in Form von Granulaten, Kapseln, Pillen, Tabletten, Filmtabletten, Dragees, Sirupen, Emulsionen, Suspensionen, Dispersionen, Aerosolen und Lösungen sowie Flüssigkeiten oder parenteral in Form von Lösungen, Emulsionen oder Suspensionen verabreicht werden. Oral zu verabreichende Präparate können einen oder mehrere Zusätze wie Süßungsmittel, Aromatisierungsmitel, Farbstoffe und Konservierungsmittel enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Ethanol, Propylenglycol, Ether des Tetrahydrofurfurylalkohols und Wasser.

Als Hilfsstoffe seien beispielsweise aufgeführt:
Wasser, nicht-toxische organische Lösungsmittel wie Paraffine (z.B. Erdölfraktionen), pfanzliche Öle (z.B. Erdnuß-/Sesam-Öl), Alkohole (z.B. Ethylalkohol, Glycerin, Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate, Dispergiermittel (z.B. Methylcellulose, Stärke und Polyvinylpyrrolidon) und Gleitmittel (z.B. Magnesiumstearat, Talkum, Stearinsäure und Natriumlaurylsulfat).

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmitel verwendet werden können.

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calziumcarbonat und Dicalziumphosphat zusammen mit verschiedenen Zuschlagstoffen, wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyoxyethylenstearat und Polyoxyethylensorbitanmonooleat und Konservierungsmitteln wie Ethylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert.

Die pharmazeutischen Präparate können den Wirkstoff in einer Menge von 0,1 bis 90 Gewichtsprozent, insbesondere 1 bis 90 Gewichtsprozent enthalten, d.h. in Mengen, die ausreichend sind, um den angegebenen Dosierungsspielraum zu erreichen, wobei der Rest ein Trägerstoff oder Zusatzstoff ist. Im Hinblick auf die Herstellung und Verabreichung werden feste Präparate wie Tabletten und Kapseln bevorzugt. Vorzugsweise enthalten die Präparate den Wirkstoff in einer Menge von 2 bis 80 mg.

Die erfindungsgemäßen Verbindungen sind nach den folgenden Verfahren erhältlich:
Aus Verbindungen der Formel III

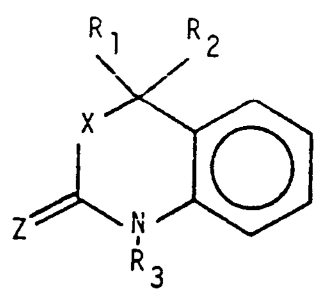

(III)

die nach literaturbekannten Verfahren zugänglich sind und in denen $R_1$, $R_2$, $R_3$, X und Z die angegebene Bedeutung haben, erhält man durch Nitrierung mit rauchender Salpetersäure (d = 1,52), anschließender Hydrierung in Gegenwart von Raney-Nickel und Einführung einer Acetyl-Schutzgruppe AC, Verbindungen der allgemeinen Formel IV,

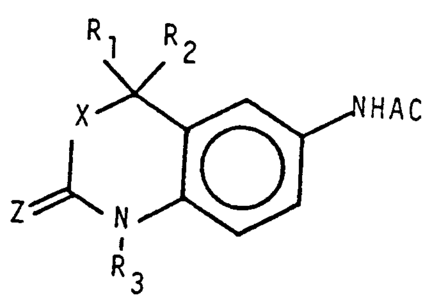

(IV)

in der $R_1$, $R_2$, $R_3$, X und Z wie eingangs definiert sind.

Durch erneute Behandlung mit rauchender Salpetersäure (d = 1,52) sind Verbindungen der allgemeinen

Formel V darstellbar,

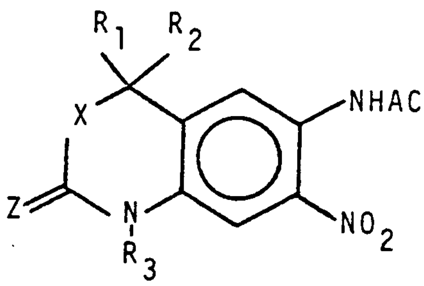

(V)

die durch anschließende Abspaltung Acetatschutzgruppe nach bekannten Methoden, Verbindungen der allgemeinen Formel VI ergeben,

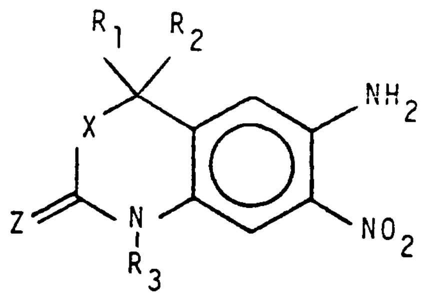

(VI)

in der $R_1$, $R_2$, $R_3$, X und Z die angegebenen Bedeutungen haben. Die Verbindungen der Formel VI werden a) entweder mit Verbindungen der Formel VII

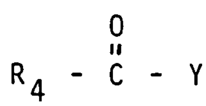

(VII)

in der $R_4$ die angegebene Bedeutung hat und Y ein Wasserstoffatom, die Hydroxygruppe, Halogen oder eine leicht abspaltbare Gruppe bedeutet, so daß mit Formel VII z.B. Aldehyde, bevorzugt Carbonsäuren oder Carbonsäurederivate gemeint sind, umgesetzt, und die Reaktionsprodukte werden anschließend unter sauren Bedingungen hydriert und zyklisiert, wodurch Verbindungen der Formel I erhalten werden, oder b) mit Raney-Nickel zu Verbindungen der allgemeinen Formel VIII hydriert,

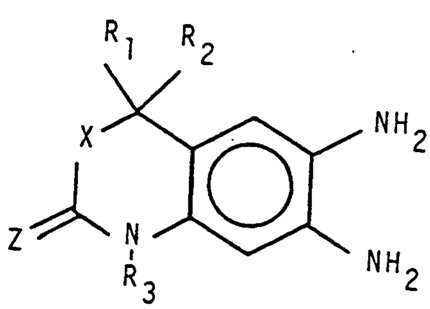

(VIII)

in der $R_1$, $R_2$, $R_3$, X und Z die angegebenen Bedeutungen haben.

Die Verbindungen der Formel VIII können durch Reaktion mit Verbindungen der Formel VII in Verbindungen der Formel IX oder deren Isomere

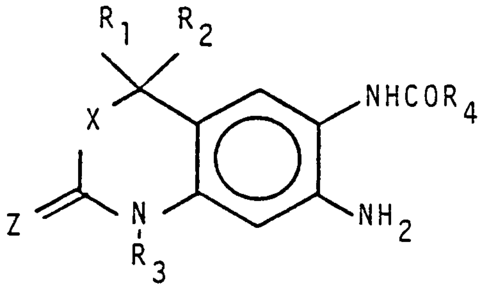

(IX)

überführt werden, in der $R_1$, $R_2$, $R_3$, $R_4$, X und Z die angegebenen Bedeutungen haben, und durch Zyklisierung in Verbindungen der allgemeinen Formel I umgewandelt werden.

Ist die im Verfahren a) genannte Verbindung VII eine Carbonsäure, so wird die Umsetzung mit Verbindungen der allgemeinen Formeln VI und VIII, vorzugsweise in aprotischen Lösungsmitteln wie Tetrahydrofuran oder Dioxan bei Temperaturen zwischen 0 ° C und Siedetemperatur des Lösungsmittels durchgeführt. Ist die Verbindung VII in Verfahren a) ein Carbonsäurederivat, z.B. ein Carbonsäurechlorid, so wird in einem inerten Lösungsmittel wie Methylenchlorid oder Pyridin bei Temperaturen zwischen 0 ° C und Siedetemperatur des Lösungsmittels gearbeitet.

Die in Verfahren a) genannte Reduktion wird vorzugsweise in einem Lösungsmittel oder Lösungsmittelgemisch wie Wasser, Methanol, Ethanol, Eisessig, Essigsäureethylester oder Dimethylformamid mit Wasserstoff in Gegenwart eines Katalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)chlorid, Natriumsulfid, Natriumhydrogensulfit oder Natriumdithionit oder mit Hydrazin in Gegenwart von Raney-Nickel bei Temperaturen zwischen 0 und 100 ° C, vorzugsweise jedoch bei Raumtemperatur, durchgeführt. Dabei entstehen meist unmittelbar die cyclisierten Verbindungen der allgemeinen Formel I.

Die Cyclisierung kann gewünschtenfalls vervollständigt werden, indem man nach der Reduktion vorzugsweise in einem Lösungsmittel oder Lösungsmittelgemisch wie Ethanol, Isopropanol, Eisessig, Benzol, Toluol, Chlorbenzol, Glycol, Ethylenglycoldimethylether, Sulfolan oder Dimethylformamid auf Temperaturen zwischen 50 und 220 ° C, vorzugsweise jedoch auf die Siedetemperatur des Reaktionsgemisches, gegebenenfalls in Gegenwart eines Kondensationsmittels wie Phosphoroxychlorid, Thionylchlorid, p-Toluolsulfonsäure, Salzsäure, Schwefelsäure, Phosphorsäure, Polyphosphorsäure oder gegebenenfalls auch in Gegenwart einer Base wie Natriumhydroxid, Natriumethylat oder Kalium-tert.-butylat erhitzt. Die Cyclisierung kann jedoch auch ohne Lösungsmittel und/oder Kondensationsmittel durchgeführt werden.

Ist die Verbindung der allgemeinen Formel VII in Verfahren b) eine Carbonsäure, so findet die Umsetzung mit Verbindungen der allgemeinen Formel V in Gegenwart eines wasserentziehenden Mittels, vorzugsweise in Polyphosphorsäure bei Temperaturen zwischen 50 und 250 ° C, vorzugsweise zwischen 100 und 200 ° C statt.

Ist die Verbindung der allgemeinen Formel VII ein Carbonsäurederivat, so findet die Umsetzung mit Verbindungen der allgemeinen Formel VIII in einem inerten Lösungsmittel, vorzugsweise in Methylenchlorid oder Pyridin statt. Zur Vervollständigung der Cyclisierung erhitzt man anschließend in einem Lösungsmittels oder Lösungsmittelgemisch wie Ethanol, Isopropanol, Eisessig, Toluol, oder Dimethylformamid auf Temperaturen zwischen 50 ° C und Siedetemperatur des Lösungsmittels.

Die Umwandlung von Verbindungen der allgemeinen Formel V, in der X eine $CH_2$-Gruppe und $R_1$ und $R_2$ Wasserstoff oder Alkyl bedeuten, zu anderen Verbindungen der Formel V, in der X eine CH-Gruppe, $R_1$ eine Valenz und $R_2$ Wasserstoff oder Alkyl bedeuten, kann durch Verwendung geeigneter Oxidationsmittel wie z. B. Mangandioxid oder Dichlordicyanbenzochinon in geeigneten Lösungsmitteln wie DMF, THF, Dioxan, bevorzugt bei den Siedetemperaturen dieser Lösungsmittel bewirkt werden. Man erhält Verbindungen der Formel X,

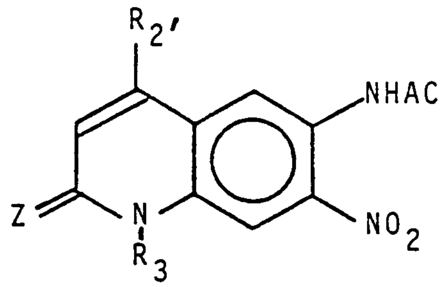

(X)

in der $R_3$, AC und Z die angegebene Bedeutung haben und $R_{2'}$ Wasserstoff oder Alkyl ist, welche in oben beschriebener Weise in Verbindungen der allgemeinen Formel XI überführt werden können,

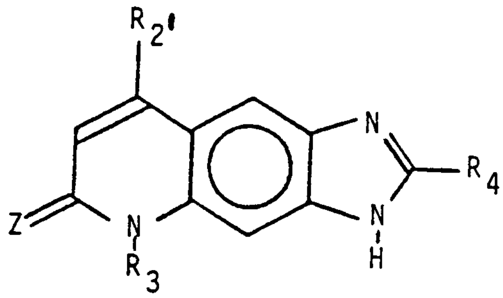

(XI)

in welcher $R_{2'}$, $R_3$, $R_4$ und Z die genannten Bedeutungen haben.

Die Umwandlung von Verbindungen der allgemeinen Formel I zu anderen Verbindungen der allgemeinen Formel I trifft zum Beispiel zu

A. für die Umsetzung einer Verbindung für die Fälle a) und a[1]), wobei $R_4$ eine Amino-, Aminoalkyl- oder cyclische Iminogruppe darstellt, oder einen mit einer Aminogruppe substituierten heterocyclischen Fünf- oder Sechsring darstellt, oder einen Phenylring der allgemeinen Formel II darstellt, in der ein oder mehrere der Substituenten $R_5$, $R_6$, $R_7$ eine Aminogruppe darstellen, und/oder in der $R_3$ ein Wasserstoffatom darstellt, mit Carbonsäuren oder mit aktivierten Carbonsäurederivaten wie Anhydriden oder Säurehalogeniden zu Formylamino- oder Alkylcarbonylaminoderivaten. Umsetzungen mit Carbonsäuren führt man vorzugsweise in Gegenwart eines wasserentziehenden Mittels wie z.B. Polyphosphorsäure oder eines mit Wasser ein azeotropes Gemisch bildenden Lösungsmittels wie Benzol oder Toluol durch. Umsetzungen mit aktivierten Carbonsäurederivaten werden vorzugsweise in inerten Lösungsmitteln wie Methylenchlorid oder Pyridin bei Temperaturen zwischen 0 ° C und 250 ° C, vorzugsweise jedoch bei der Siedetemperatur des Lösungsmittels durchgeführt.

B. für die Umsetzung von Verbindungen der Fälle a) und a[1]), wobei $R_4$ eine Amino-, Aminoalkyl- oder cyclische Iminogruppe darstellt, oder $R_4$ einen mit einer Aminogruppe substituierten heterocyclischen Fünf- oder Sechsring darstellt, wie er eingangs definiert ist, oder $R_4$ einen Phenylring der allgemeinen Formel II darstellt, in welcher einer der Substituenten $R_5$, $R_6$, $R_7$ eine Amino-, N-Alkylamino- oder Hydroxygruppe darstellt, mit einer Sulfonsäure der allgemeinen Formel XII

$R_8$-$SO_2OH$ (XII)

in der $R_8$ eine Alkylgruppe mit 1 - 3 Kohlenstoffatomen oder eine Trifluormethylgruppe darstellt oder mit einem reaktionsfähigen Derivat hiervon, zu Verbindungen von a) und a[1]), worin die besagten Amino-, Aminoalkyl-, cyclischen Imino-, N-Alkylamino- oder Hydroxygruppen sulfoniert sind. Die Umsetzung wird zweckmäßigerweise in einem Lösungsmittel oder Lösungsmittelgemisch wie Methylenchlorid, Ether, Tetrahydrofuran, Dioxan oder Benzol gegebenenfalls in Gegenwart eines säurebindenen Mittels wie Natriumcarbonat, Triethylamin oder Pyridin, wobei die beiden letzten gleichzeitig auch als Lösungsmittel verwendet werden können, in Gegenwart eines die Säure aktivierenden oder wasserentziehenden Mittels wie Thionylchlorid oder Phosphorpentachlorid, vorzugsweise jedoch mit einem reaktionsfähigen Derivat einer Verbindung der allgemeinen Formel XII, z.B. mit deren Anhydrid oder Halogenid wie Methansulfonsäurechlorid oder Ethansulfonsäurechlorid, vorzugsweise bei Temperaturen zwischen 0 und 100 ° C, z.B. bei Temperaturen zwischen Raumtemperatur und 50 ° C, durchgeführt.

C. für die Umwandlung von Verbindungen von a) und $a_1$), wobei $R_4$ eine Phenylgruppe der allgemeinen Formel II darstellt, wobei einer der Substituenten $R_5$, $R_6$, $R_7$ eine Alkylmercapto- oder Alkylsulfenylmethylgruppe mit 1 - 3 Kohlenstoffatomen im Alkylteil ist, zu Verbindungen von a) und $a^1$), wobei $R_1$ eine Phenylgruppe und einer der Substiuenten $R_5$, $R_6$, $R_7$ eine Alkylsulfinyl-, Alkylsulfonyl-, Alkylsulfinylmethyl- oder Alkylsulfonylmethylgruppe darstellt.

Diese Oxidation wird vorzugsweise in einem Lösungsmittel oder Lösungsmittelgemisch, z.B. in Wasser, Wasser/Pyridin, Aceton, Eisessig, verdünnter Schwefelsäure oder Trifluoressigsäure, je nach dem verwendeten Oxidationsmitel zweckmäßigerweise bei Temperaturen zwischen -80 und 100°C durchgeführt. Zur Herstellung einer Alkylsulfinyl- oder Alkylsulfinylmethylverbindung der allgemeinen Formel I wird die Oxidation zweckmäßigerweise mit einem Äquivalent des verwendeten Oxidationsmittel durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20°C bis 60°C, mit Natriummetaperjodat in wäßrigem Methanol oder Ethanol bei -15 bis 25°C, mit Brom in Eisessig oder wäßriger Essigsäure, mit N-Brom-succinimid in Ethanol, mit tert.-Butylhypochlorit in Methanol bei -80°C bis -30°C, mit Jodbenzodichlorid in wäßrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C; der hierbei erhaltene Thioether-Chlor-Komplex wird zweckmäßigerwiese mit wäßrigem Ethanol hydrolysiert.

Zur Herstellung einer Alkylsulfonylverbindung von a) und $a^1$) wird die Oxidation zweckmäßigerweise mit einem bzw. mit zwei oder mehr Äquivalenten des verwendeten Oxidationsmittels durchgeführt, z.B. Wasserstoffperoxid in Eisessig, Trifluoressigsäure oder in Ameisensäure bei 20 bis 100°C oder in Aceton bei 0 bis 60°C, mit einer Persäure wie Perameisensäure, oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

D. für die Umwandlung von Verbindungen von a) und $a^1$), worin $R_4$ eine Phenylgruppe der allgemeinen Formel II darstellt, wobei einer der Substituenten $R_5$, $R_6$, $R_7$ eine Carboxyl- oder Hydroxylsulfonylgrupe darstellt, zu Verbindungen von a) und $a^1$), worin einer der Substituenten $R_5$, $R_6$ und $R_7$ eine durch eine Amino-, Alkylamino- oder Dialkylaminogruppe substituierte Carbonyl- oder Sulfonylgruppe darstellt. Dies geschieht durch Umsetzung mit einem Amin $HNR_9R_{10}$, wobei $R_9$ und $R_{10}$ gleich oder verschieden sein können und Wasserstoff oder $C_1$ - $C_5$ Alkylgruppen bedeuten, oder mit einem reaktionsfähigen Derivat hiervon. Vorteilhaft ist es, die Carboxylgruppe oder Hydroxysulfonylgruppe in ein reaktionsfähiges Derivat, z.B. in einen Ester oder ein Säurechlorid zu verwandeln und dann mit dem Amin $HNR_9R_{10}$ umzusetzen.

Die Umsetzung wird zweckmäßigerweise in einem Lösungsmittel oder Lösungsmittelgemisch wie Methylenchlorid, Ethanol Choroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäurethylester, Thionylchlorid, Phosphortrichlorid, Phosphorpentoxid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels durchgeführt werden; desweiteren kann während der Umsetzung entstehendes Wasser durch azeotrope Destillation, z.B. durch Erhitzen mit Toluol am Wasserabschneider, oder durch Zugabe eines Trockenmittels wie Magnesiumsulfat oder Molekularsieb abgetrennt werden.

Besonders vorteilhaft wird jedoch die Umsetzung in einem entsprechenden Halogenid, z.B. dem Carbonsäure- oder Sulfonsäurechlorid, und einem entsprechenden Amin, wobei dieses gleichzeitig als Lösungsmittels dienen kann, und bei Temperaturen zwischen 0 und 50°C durchgeführt.

E. für die Umwandlung von Verbindungen von a) und $a^1$), worin $R_1$ einen Phenylring der allgemeinen Formel II darstellt und einer der Substituierten $R_5$, $R_6$ oder $R_7$ die Cyangruppe darstellt und/oder in der $R_3$ einen Alkanoylrest darstellt, zu Verbindungen der allgemeinen Formel I, in welcher $R_1$ einen Phenylring der allgemeinen Formel II darstellt und einer der Substituenten $R_5$, $R_6$ oder $R_7$ eine Alkoxycarbonylgruppe-, eine Aminocarbonylgruppe oder eine Carboxylgruppe bedeutet, und/oder in der $R_3$ ein Wasserstoffatom darstellt.

Diese Alkoholyse und/oder Hydrolyse wird entweder in Gegenwart einer Säure wie Salzsäure,

Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Ethanol, Wasser/Ethanol, Wasser/Isopropanol oder Wasser/Dioxan bei Temperaturen zwischen -10 und 120 °C, z.B. bei Temperaturen zwischen Raumtemperatur und der Siedetemperatur des Reaktionsgemisches, durchgeführt.

F. für die Alkylierung von Verbindungen von a) und a[1]), wobei $R_4$ einen Phenylring der allgemeinen Formel II darstellt, in der einer der Substituenten $R_5$, $R_6$ oder $R_7$ eine Hydroxy- oder Mercaptogruppe bedeutet, oder in welcher $R_4$ einen mit einer Hydroxy- oder Mercaptogruppe substituierten heterocyclischen Ring darstellt. Dabei entstehen die entsprechenden Alkylmercapto oder Alkyloxyverbindungen.

Die Reaktionen werden vorzugsweise in einem Lösungsmittel wie Aceton, Ether, Benzol, Toluol oder Dimethylformamid bei Temperaturen zwischen -30 °C und +100 °C, vorzugsweise bei Raumtemperatur in Gegenwart einer Base wie Kaliumcarbonat oder Natriumhydrid und eines Alkylierungsmittels wie Alkylhalogeniden oder Alkylsulfaten durchgeführt.

G. für die Reduktion von Verbindungen von a) und a[1]), wobei $R_4$ einen Pyridinring darstellt zu Verbindungen von a) und a[1]), worin $R_4$ den Piperidinring bedeutet. Diese Reduktionen führt man vorzugsweise im alkoholischen Medium in Gegenwart eines Katalysators wie Platin oder Palladium mittels Wasserstoff bei Normaldruck oder leicht erhöhtem Druck und einer Temperatur zwischen Raumtemperatur und 60 °C durch.

H. für die Oxidation eines Fünf- oder Sechsringes mit einem oder mehreren Stickstoffatomen zu den entsprechenden N-Oxiden. Die Oxidation wird zweckmäßig mit einem oder mehreren Äquivalenten des verwendeten Oxidationsmittels durchgeführt, z.B. mit Wasserstoffperoxid in Eisessig, Trifluoressigsäure oder in Ameisensäure bei 20-100 °C oder in Aceton bei 0-60 °C, mit einer Persäure wie Perameisensäure oder m-Chlorperbenzoesäure in Eisessig, Trifluoressigsäure, Methylenchlorid oder Chloroform bei Temperaturen zwischen 0 und 60 °C.

Die Ausgangsverbindungen der Formeln III bis XII sind bekannt oder nach bekannten Verfahren erhältlich.

Die Verbindungen der allgemeinen Formel I können sowohl Basen als auch Säuren bzw. amphoter sein und daher in der Form ihrer Salze oder Säureadditionssalze aus den Reaktionsgemischen isoliert werden. Sie lassen sich als Basen mit geeigneten anorganischen oder organischen Säuren nach bekannten Verfahren in Salze überführen oder bilden als Säuren mit Basen Salze.

Bevorzugt werden physiologisch verträgliche Salze oder Säureadditionssalze. Hierfür sind als anorganische Säuren beispielsweise Halogenwasserstoffsäuren, zum Beispiel Salzsäure oder Schwefelsäure, und als organische Säuren zum Beispiel Fumarsäure, Maleinsäure, Zitronensäure und Weinsäure geeignet. Zur Herstellung wird die heiße alkoholische Lösung der Base mit der alkoholischen Lösung einer geeigneten Säure versetzt, und man erhält nach Etherzusatz das Salz. Bevorzugte Salze sind die Alkali-, Erdalkali- und Ammoniumsalze der Verbindungen der Formel I, die mit den entsprechenden Basen, insbesondere Natrium-, Kalium- oder Ammoniumhydroxid, erhalten werden.

Diastereoisomere können in bekannter Weise aufgrund der physikalisch-chemischen Unterschiede ihrer Bestandteile in ihre racemischen Modifikationen getrennt werden. Racemate können nach bekannten Methoden getrennt werden, beispielsweise durch Umkristallisieren in optisch aktiven Lösungsmitteln, durch Mikroorganismen oder Reaktion mit einer optisch aktiven Säure oder Base, die mit der racemischen Verbindung ein Salz bildet, Trennung der Diastereoisomeren durch fraktionierte Kristallisation und Freisetzung der Enantiomeren durch geeignete Mittel. Besonders geeignete optisch aktive Säuren sind beispielsweise die D- und L-Formen der Weinsäure, Ditoluoylweinsäure, Äpfelsäure, Mandelsäure, Kamphersulfonsäure oder Pyrrolidon-carbonsäure. Geeignete optisch aktive Basen sind alpha-Phenyläthylamin, Menthylamin, Ephedrin, Brucin und Chinin. Vorteilhafterweise wird der aktivere der Antipoden isoliert. Gemäß der Erfindung ist es jedoch auch möglich, die reinen Enantiomeren durch asymmetrische Synthese zu erhalten.

Die folgenden Beispiele dienen zur Erläuterung der Erfindung.

Beispiel 1

2-Cyanamino-6,7-dihydro-7,7-dimethyl-3H,5H-pyrrolo(2,3-f)benzimidazol-6-on

1 g (5,23 mMol) 5,6-Diamino-3,3-indolin-2-on wird zusammen mit 1,24 g (5,23 mMol) N-Cyanodiphenoxyimidocarbonat in 20 ml Methanol sechs Stunden am Rückfluß erhitzt. Das Reaktionsprodukt wird abgesaugt, mit Isopropanol gewaschen und getrocknet.

Ausbeute: 0,57 g (44% d. Th.), Schmelzpunkt über 300 °C $C_{12}H_{11}N_5O$ (241,26)

| | | | |
|---|---|---|---|
| Ber. | C 59.74 | H 4.60 | N 29.03 |
| Gef. | C 59.21 | H 4.73 | N 28.94 |

Beispiel 2

2-Cyanamino-8,8-dimethyl-8-hydro-5H-imidazol(4,5-g) (3,1)-benzoxazin-6-on

1 g (4,8 mMol) 6,7-Diamino-4,4-dimethyl-4H-(3,1)-benzoxazin-2-on wird mit 1,14g (4.8 mmol) N-Cyanodiphenoxyimido-Carbonat, wie in Beispiel 1 beschrieben, umgesetzt und aufgearbeitet.
Ausbeute: 0,59 g (48% d. Th.), Schmelzpunkt über 300 ° C $C_{12}H_{11}N_5O_2$ (257,61)

| | | | |
|---|---|---|---|
| Ber. | C 55.94 | H 4.30 | N 27.33 |
| Gef. | C 55.51 | H 4.20 | N 27.15 |

Beispiel 3

2-Cyanamino-7,8-dihydro-3H,5H-imidazo(4,5-g)chinolin-6-on

1 g (5,6 mMol) 6,7-Diamino-3,4-dihydro-1H-chinolin-2-on wird zusammen mit 1,33 g (5,6 mMol) N-Cyanodiphenoxyimidocarbonat, wie in Beispiel 1 beschrieben, umgesetzt und aufgearbeitet.
Ausbeute: 0,65 g (50% d. Th.), Schmelzpunkt über 300 ° C $C_{11}H_9N_5O$ (211,58)

| | | | |
|---|---|---|---|
| Ber. | C 62.44 | H 4.29 | N 33.27 |
| Gef. | C 62.49 | H 4.20 | N 33.19 |

Beispiel 4

2-((4-Difluormethoxy)-3-pyridyl)-6,7-dihydro-7,7-dimethyl-3H,5H-pyrrolo(2,3-f)-benzimidazol-6-on

In eine Suspension von 0,95 g (5 mMol) 5,6-Diamino-3,3-indolin-2-on in 5 ml Dichlormethan und 1,75 ml Triethylamin werden 1,14 g (5,5 mMol) 2-Difluormethoxynikotinsäurechlorid in 5 ml Dichlormethan gelöst getropft.

Es wird 8 Stunden bei Raumtemperatur gerührt. Anschließend engt man im Vakuum bis zur Trockne ein und versetzt den Rückstand mit 100 ml Ethanol und 10 ml konzentrierter Salzsäure und erhitzt 4 Stunden am Rückfluß. Man engt im Vakuum bis zur Trockne ein, versetzt mit Ammoniak bis zur alkalischen Reaktion und saugt den Rückstand ab.

Es folgt eine säulenchromatographische Reinigung an Kieselgel. (Laufmittel: Dichlormethan/Methanol 90:10 v/v)
Ausbeute: 0,60 g (35% d. Th.), Schmelzpunkt über 300 ° C. $C_{17}H_{14}F_2N_4O_2$ (344.33)

| | | | | |
|---|---|---|---|---|
| Ber. | C 59.30 | H 4.10 | F 11.04 | N 16.27 |
| Gef. | C 58.30 | H 4.25 | F 10.61 | N 15.89 |

Beispiel 5

2-(4-Pyridyl)-3H,5H-imidazo(4,5-g)chinolin-6-on

1 g (5.6 mMol) 6,7-Diamino-(4,5-g)-chinolin-2-on werden in 10 ml Dichlormethan gelöst und mit 2 g (20 mMol) Triethylamin versetzt. In diese Lösung tropft man bei 25 ° C 1.06 g (6 mMol) Isonikotinsäurechlorid, gelöst in 10 ml Dichlormethan. Die Mischung wird 8 Stunden bei Raumtemperatur gerührt, eingeengt und

mit Wasser versetzt. Der Rückstand wird in 50 ml Ethanol aufgenommen, mit 20 ml konzentrierter Salzsäure ca. 4 Stunden am Rückfluß gehalten, eingeengt und mit Ammoniak neutral gestellt. Der Rückstand wird abgesaugt und aus Ethanol umkristallisiert.
Ausbeute: 0,8 g (56% d. Th.), Schmelzpunkt über 300 ° C $C_{15}H_{12}N_4O$ (264.29)

| | | | |
|---|---|---|---|
| Ber. | C 68.17 | H 4.58 | N 21.20 |
| Gef. | C 68.00 | H 4.62 | N 21.14 |

Beispiel 6

Herstellung von Tabletten und Kapseln

Tabletten und Kapseln, welche die nachstehend angegebenen Bestandteile enthalten, werden nach bekannten Arbeitsweisen hergestellt. Diese sind für die Behandlung der vorstehend genannten Krankheiten, insbesondere der Herzinsuffizienz in Dosierungsmengen von jeweils einer Tablette oder Kapsel einmal bis viermal täglich geeignet

| Bestandteile | Gewicht Tablette | (mg) Kapsel |
|---|---|---|
| 2-Cyanamino-6,7-dihydro-7,7-dimethyl-3H,5H-pyrrolo-(2,3-f)benzimidazol-6-on | 7 | 4 |
| Tragacanth | 10 | - |
| Lactose | 247,5 | 300 |
| Maisstärke | 25 | - |
| Talk | 15 | - |
| Magnesiumstearat | 2,5 | - |

Beispiel 7

Herstellung von Ampullen

Ampullen, die die im folgenden genannten Bestandteile enthalten, lassen sich in bekannter Weise herstellen. Wirkstoff und Natriumchlorid werden in Wasser gelöst und unter Stickstoff in Glasampullen abgefüllt.

| | |
|---|---|
| Natriumchlorid | 18 mg |
| dest. Wasser ad | 2,0 ml |
| 2-Cyanamino-6,7-dihydro-7,7-dimethyl-3H,5H-pyrrolo(2,3-f)-benzimidazol-6-on-fumarat | 1 mg |

Beispiel 8

Mit den angegebenen Bestandteilen (Gewichtsmengen) wird eine Fettsalbe hergestellt:
Weißes Vaselin 76%
dickflüssiges Paraffin 15%
Glycerin 2%
Emulgator (Polyoxyethylen-40-stearat) 2%
2-((4-Difluormethoxy)-3-pyridyl)-6,7-dihydro-7,7-dimethyl-3H,5H-pyrrolo(2,3-f)-benzimidazol-6-on 5%
Vaselin, Paraffin, Glycerin und der Emulgator werden auf 80 °C erhitzt. Dann wird der Wirkstoff zugegeben. Es wird bis zum Lösen gerührt und das Rühren bis zum Festwerden der Masse (salbenartige Konsistenz) fortgesetzt.

Die Salbe wird dreimal täglich angewendet.

Versuchsbericht

Die neuen Verbindungen der allgemeinen Formel I wurden im Hinblick auf ihre kardiovaskulären Eigenschaften untersucht.

Beispielsweise wurde die Verbindung 2-Cyanamino-6,7-dihydro-7,7-dimethyl-3H,5H-pyrrolo(2,3-f)-benzimidazol-6-on wie folgt untersucht:
Die Untersuchungen wurden mit 2,5-3,5 kg schweren Katzen durchgeführt, die mit Pentobarbital-Natrium (40 mg/kg i.p.) narkotisiert waren. Die Tiere wurden künstlich beatmet.

Für die Erfassung inotroper und chronotroper Wirkungen wurde mit einem Tipkatheter (Millar PC-350) der Druck in der linken Herzkammer gemessen. Daraus wurde der Kontraktilitätsparameter dp/dtmax mittels eines Analogdifferenzierers und die Herzfrequenz (HF) mittels eines Frequenzzählers registriert.

Der arterielle Blutdruck wurde in der Arteria femoralis mit einem Statham-Druckwandler (P 23 Db) gemessen. Die zu untersuchende Substanz wurde in die Vena jugularis injiziert. Als Lösungsmittel diente Dimethylsulfoxid/Polyethylenglykol. Die Substanz wurde an jeweils 2-4 Tieren in den Dosen 0,001, 0,01, 0,1, 1 mg/kg i.v. untersucht.

| Dosis (mg/kg) | dp/dtmax (%) | HF (5) | art.Mitteldruck |
|---|---|---|---|
| 0,001 | + 10 | 0 | 0 |
| 0,01 | + 37 | 0 | 0 |
| 0,1 | + 56 | + 3 | 0 |
| 1 | + 67 | + 8 | - 15 |

Toxische Nebenwirkungen wurden nicht beobachtet.

**Patentansprüche**

**1.** Verbindungen der Formel I

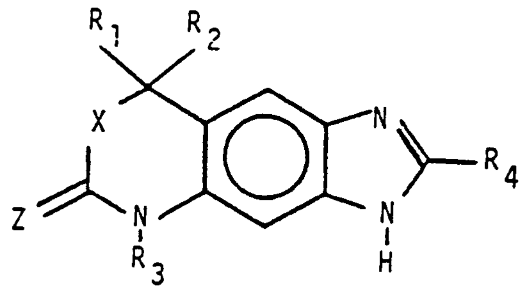

(I)

in der
X die Bedeutung $X_a$, $X_aI$, $X_b$ oder $X_c$ haben kann, und in der im Fall

a), in dem X die Bedeutung $X_a$ hat,
$X_a$ die Methylengruppe $-CH_2-$ bedeutet, und
$R_1$ die Bedeutung $R_{1a}$ hat und
$R_{1a}$ Wasserstoff, $C_{1-6}$-Alkyl, $C_{2-6}$-Alkenyl oder $C_{3-7}$-Cycloalkyl ist und
$R_2$ die Bedeutung $R_{2a}$ hat und
$R_{2a}$ Wasserstoff, $C_{1-6}$-Alkyl, $C_{2-6}$-Alkenyl oder $C_{1-6}$-Hydroxyalkyl bedeutet, wobei $R_{1a}$ und $R_{2a}$ gleich oder verschieden sein können, oder
$R_{1a}$ und $R_{2a}$ zusammen mit dem von ihnen eingeschlossenen Kohlenstoffatom $C_{3-7}$-Spiroalkyl bedeuten,
$R^3$ Wasserstoff oder $C_{1-6}$-Alkyl bedeutet,
$R_4$ die Bedeutung $R_{4a}$ hat und
$R_{4a}$ die Cyanaminogruppe, die 4-Difluormethoxy-3-pyridylgruppe oder eine $C_{1-2}$-Nitroalkylgruppe ist und
Z Sauerstoff oder Schwefel bedeutet,
und in der im Fall
$a^1$), in dem X die Bedeutung $X_aI$ hat,
$X_aI$ zusammen mit $R_1$, das in diesem Fall die Bedeutung
$R_{1a}I$ hat, die Gruppe -CH= bedeutet und
$R_2$ die Bedeutung $R_{2a}I$ hat und
$R_{2a}I$ Wasserstoff oder $C_{1-6}$-Alkyl ist,
$R_3$ Wasserstoff oder $C_{1-6}$-Alkyl bedeutet,
$R_4$ die Bedeutung $R_{4a}I$ hat und
$R_{4a}I$ die Cyanaminogruppe, die 4-Difluormethoxy-3-pyridylgruppe, eine $C_{1-2}$-Nitroalkylgruppe, Wasserstoff, die Hydroxygruppe, eine Mercapto-, $C_{1-6}$-Alkylmercapto-, eine Amino-, Pyridylcarbonylamino-, $C_{1-6}$-Alkylcarbonylamino-, eine $C_{1-6}$-Alkyl-, $C_{3-7}$-Cycloalkyl-, $C_{2-6}$-Alkenyl-, $C_{3-7}$-Cycloalkenyl-, $C_{2-6}$-Alkinyl-, $C_{1-6}$-Halogenalkylgruppe oder einen Phenylring der allgemeinen Formel II

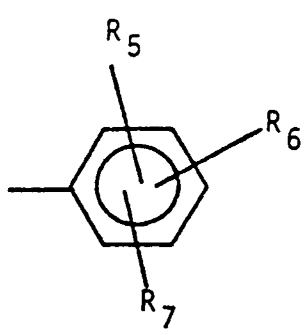

(II)

bedeutet, wobei $R_5$, $R_6$, $R_7$ gleich oder verschieden sein können und jeweils Wasserstoff, eine $C_{1-6}$-Alkansulfonyloxy-, Trifluormethansulfonyloxy-, $C_{1-6}$-Alkansulfonylamino-, Trifluormethansulfonylamino-, N-$C_{1-6}$-Alkyl-$C_{1-6}$-alkylsulfonylamino-, N-$C_{1-6}$-Alkyltrifluormethansulfonylamino-, $C_{1-6}$-Alkylsulfenylmethyl-, $C_{1-6}$-Alkylsulfinylmethyl- oder $C_{1-6}$-Alkylsulfonylmethylgruppe, eine durch eine Hydroxy-, $C_{1-6}$-Alkoxy-, Amino-, $C_{1-6}$-Alkylamino- oder

$C_{2-12}$-Dialkylaminogruppe substituierte Carbonylgruppe, eine durch eine Amino-, $C_{1-6}$-Alkylamino-, $C_{2-12}$-Dialkylamino- oder cyclische Iminogruppe substituierte Sulfonylgruppe, wobei eine Methylengruppe in 4-Stellung durch ein Schwefel- oder Sauerstoffatom ersetzt sein kann, eine $C_{1-6}$-Alkylcarbonylamino-, Aminocarbonylamino- oder $C_{1-6}$-Alkylaminocarbonylaminogruppe, eine $C_{1-6}$-Alkylmercapto-, $C_{1-6}$-Alkylsulfinyl- oder $C_{1-6}$-Alkylsulfonylgruppe, eine Nitro-, Halogen-, Amino-, Hydroxy-, $C_{1-6}$-Alkyl-, $C_{1-6}$-Alkoxy-,$C_{1-6}$-Alkenyloxy-, $C_{1-6}$-Alkinyloxy-, Cyan-$C_{1-6}$-alkyloxy-, Carboxy-$C_{1-6}$-alkoxy, $C_{1-6}$-Alkoxycarbonyl-$C_{1-6}$-alkyloxy-, $C_{2-12}$-Dialkylamino-, 1-Imidazolyl-, Trifluormethyl- oder Cyangruppe sein können,
oder $R_{4a}$l einen Naphthylrest bedeutet
oder $R_{4a}$l einen gesättigten oder ungesättigten heterocyclischen Fünfring mit 1-4 Heteroatomen oder einen gesättigten oder ungesättigten heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere $C_{1-6}$-Alkyl-, $C_{1-6}$-Alkoxy-, $C_{1-6}$-Alkylmercapto-, Hydroxy-, Nitro-, Amino-, Halogen- oder Cyangruppen substituiert oder mit einem Phenylring zu einem Bicyclus kondensiert sein können und
Z Sauerstoff oder Schwefel bedeutet,
und in der im Fall
b), in dem X die Bedeutung $X_b$ hat,
$X_b$ Sauerstoff bedeutet und
$R_1$ die Bedeutung $R_{1b}$ und $R_2$ die Bedeutung $R_{2b}$ hat und
$R_{1b}$ und $R_{2b}$, die gleich oder verschieden sein können, jeweils für Wasserstoff oder $C_{1-6}$-Alkyl stehen,
$R_3$ Wasserstoff oder $C_{1-6}$-Alkyl bedeutet,
$R_4$ die Bedeutung $R_{4b}$ hat und
$R_{4b}$ die Cyanaminogruppe, die 4-Difluormethoxy-3-pyridylgruppe oder eine $C_{1-2}$-Nitroalkylgruppe ist und
Z Sauerstoff oder Schwefel bedeutet,
und in der im Fall
c), in dem X die Bedeutung $X_c$ hat, $X_c$ eine Bindung bedeutet und
$R_1$ die Bedeutung $R_{1c}$ hat und
$R_{1c}$ Wasserstoff, $C_{1-6}$-Alkyl, $C_{2-6}$-Alkenyl oder $C_{3-7}$-Cycloalkyl ist und
$R_2$ die Bedeutung $R_{2c}$ hat und
$R_{2c}$ Wasserstoff, $C_{1-6}$-Alkyl, $C_{2-6}$-Alkenyl oder $C_{1-6}$-Hydroxyalkyl bedeutet, wobei $R_{1c}$ und $R_{2c}$ gleich oder verschieden sein können, oder
$R_{1c}$ und $R_{2c}$ zusammen mit dem von ihnen eingeschlossenen Kohlenstoffatom $C_{3-7}$-Spiroalkyl bedeuten,
$R_3$ Wasserstoff oder $C_{1-6}$-Alkyl bedeutet,
$R_4$ die Bedeutung $R_{4c}$ hat und
$R_{4c}$ die Cyanaminogruppe, die 4-Difluormethoxy-3-pyridylgruppe oder eine $C_{1-2}$-Nitroalkylgruppe ist und
Z Sauerstoff oder Schwefel bedeutet,
sowie deren Salze und Säureadditionssalze, Tautomere und optische Isomere.

**2.** 2-Cyanamino-8,8-dimethyl-8-hydro-5H-imidazo(4,5-g)(3,1) benzoxazin-6-on gemäß Anspruch 1.

**3.** 8,8-Dimethyl-2-(4-difluormethoxy-3-pyridyl)-8-hydro-5H-imidazo(4,5-g)(3,1)benzoxazin-6-on gemäß Anspruch 1.

**4.** 2-Cyanamino-7,8-dihydro-3H,5H-imidazo(4,5-g)chinolin-6-on gemäß Anspruch 1.

**5.** 2-((4-Difluormethoxy)-3-pyridyl)-6,7-dihydro-7,7-dimethyl-3H,5H-pyrrolo(2,3-f)-benzimidazol-6-on gemäß Anspruch 1.

**6.** 2-Cyanamino-6,7-dihydro-7,7-dimethyl-3H,5H-pyrrolo(2,3-f)benzimidazol-6-on gemäß Anspruch 1.

**7.** Verfahren zur Herstellung der Verbindungen der Formel I, dadurch gekennzeichnet, daß man Verbindungen der allgemeinen Formel VI,

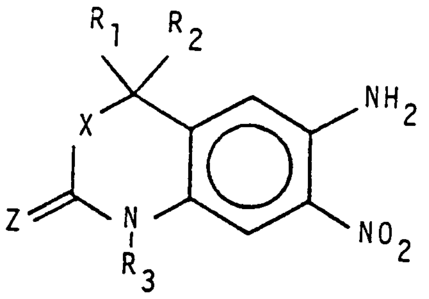

(VI)

in der $R_1$, $R_2$, $R_3$, X und Z die angegebenen Bedeutungen haben, entweder mit Verbindungen der Formel VII

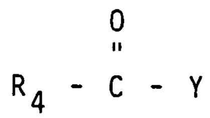

(VII)

in der $R_4$ die angegebene Bedeutung hat und Y Wasserstoff, Hydroxy, Halogen oder eine leicht abspaltbare Gruppe darstellt, umsetzt, und die Reaktionsprodukte anschließend unter sauren Bedingungen hydriert und zyklisiert, wodurch Verbindungen der Formel I erhalten werden, oder mit Raney-Nickel zu Verbindungen der allgemeinen Formel VIII hydriert,

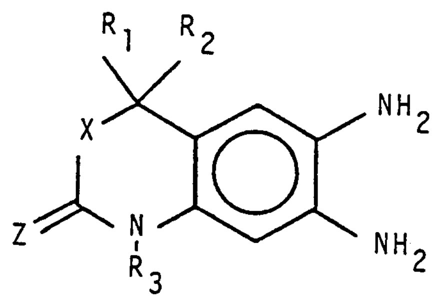

(VIII)

in der $R_1$, $R_2$, $R_3$, X und Z die angegebenen Bedeutungen haben, oder die Verbindungen der Formel VIII durch Reaktion mit Verbindungen der Formel VII in Verbindungen der Formel IX oder deren Isomere

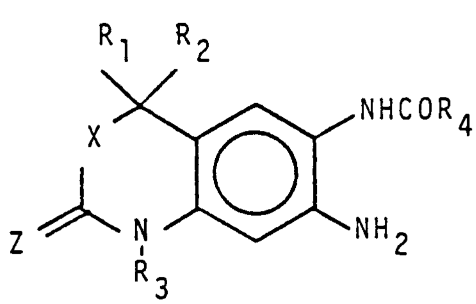

überführt, in der $R_1$, $R_2$, $R_3$, $R_4$, X und Z die angegebenen Bedeutungen haben und durch Zyklisierung in Verbindungen der allgemeinen Formel I umwandelt.

**8.** Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält sowie topische Zubereitung mit diesen Verbindungen.

**9.** Verwendung der Verbindungen gemäß Anspruch 1 als PDE-III-und-IV-Hemmstoffe.

**Patentansprüche für folgenden Vertragsstaat: ES**

**1.** Verfahren zur Herstellung von Verbindungen der Formel I

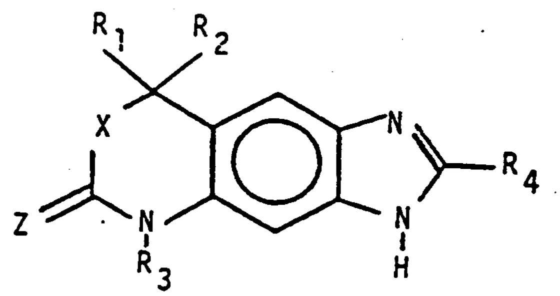

(I)

in der
X die Bedeutung $X_a$, $X_aI$, $X_b$ oder $X_c$ haben kann, und in der im Fall
a), in dem X die Bedeutung $X_a$ hat,
$X_a$ die Methylengruppe $-CH_2-$ bedeutet, und
$R_1$ die Bedeutung $R_{1a}$ hat und
$R_{1a}$ Wasserstoff, $C_{1-6}$-Alkyl, $C_{2-6}$-Alkenyl oder $C_{3-7}$-Cycloalkyl ist und
$R_2$ die Bedeutung $R_{2a}$ hat und
$R_{2a}$ Wasserstoff, $C_{1-6}$-Alkyl, $C_{2-6}$-Alkenyl oder $C_{1-6}$-Hydroxyalkyl bedeutet, wobei $R_{1a}$ und $R_{2a}$ gleich oder verschieden sein können, oder
$R_{1a}$ und $R_{2a}$ zusammen mit dem von ihnen eingeschlossenen Kohlenstoffatom $C_{3-7}$-Spiroalkyl bedeuten,
$R^3$ Wasserstoff oder $C_{1-6}$-Alkyl bedeutet,
$R_4$ die Bedeutung $R_{4a}$ hat und
$R_{4a}$ die Cyanaminogruppe, die 4-Difluormethoxy-3-pyridylgruppe oder eine $C_{1-2}$-Nitroalkylgruppe ist und
Z Sauerstoff oder Schwefel bedeutet,
und in der im Fall
a[1]), in dem X die Bedeutung $X_aI$ hat,
$X_aI$ zusammen mit $R_1$, das in diesem Fall die Bedeutung
$R_{1a}I$ hat, die Gruppe -CH= bedeutet und
$R_2$ die Bedeutung $R_{2a}I$ hat und
$R_{2a}I$ Wasserstoff oder $C_{1-6}$-Alkyl ist,
$R_3$ Wasserstoff oder $C_{1-6}$-Alkyl bedeutet;
$R_4$ die Bedeutung $R_{4a}I$ hat und
$R_{4a}I$ die Cyanaminogruppe, die 4-Difluormethoxy-3-pyridylgruppe, eine $C_{1-2}$-Nitroalkylgruppe, Wasserstoff, die Hydroxygruppe, eine Mercapto-, $C_{1-6}$-Alkylmercapto-, eine Amino-, Pyridylcarbonylamino-, $C_{1-6}$-Alkylcarbonylamino-, eine $C_{1-6}$-Alkyl-, $C_{3-7}$-Cycloalkyl-, $C_{2-6}$-Alkenyl-, $C_{3-7}$-Cycloalkenyl-, $C_{2-6}$-Alkinyl-, $C_{1-6}$-Halogenalkylgruppe oder einen Phenylring der allgemeinen Formel II

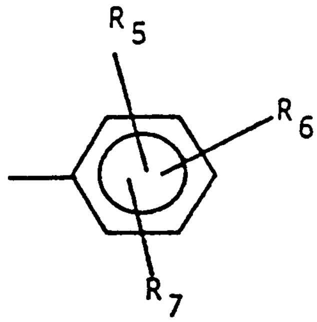

(II)

bedeutet, wobei $R_5$, $R_6$, $R_7$ gleich oder verschieden sein können und jeweils Wasserstoff, eine $C_{1-6}$-Alkansulfonyloxy-, Trifluormethansulfonyloxy, $C_{1-6}$-Alkansulfonylamino-, Trifluormethansulfonylamino-, N-$C_{1-6}$-Alkyl-$C_{1-6}$-alkylsulfonylamino-, N-$C_{1-6}$-Alkyltrifluormethansulfonylamino-, $C_{1-6}$-Alkylsulfenylmethyl-, $C_{1-6}$-Alkylsulfinylmethyl- oder $C_{1-6}$-Alkylsulfonylmethylgruppe, eine durch eine Hydroxy-, $C_{1-6}$-Alkoxy-, Amino-, $C_{1-6}$-Alkylamino- oder $C_{2-12}$-Dialkylaminogruppe substituierte Carbonylgruppe, eine durch eine Amino-; $C_{1-6}$-Alkylamino-, $C_{2-12}$-Dialkylamino- oder cyclische Iminogruppe substituierte Sulfonylgruppe, wobei eine Methylengruppe in 4-Stellung durch ein Schwefel- oder Sauerstoffatom ersetzt sein kann, eine $C_{1-6}$-Alkylcarbonylamino-, Aminocarbonylamino- oder $C_{1-6}$-Alkylaminocarbonylaminogruppe, eine $C_{1-6}$-Alkylmercapto, $C_{1-6}$-Alkylsulfinyl- oder $C_{1-6}$-Alkylsulfonylgruppe, eine Nitro-, Halogen-, Amino-, Hydroxy-, $C_{1-6}$-Alkyl-, $C_{1-6}$-Alkoxy-,$C_{1-6}$-Alkenyloxy-, $C_{1-6}$-Alkinyloxy-, Cyan-$C_{1-6}$-alkyloxy-, Carboxy-$C_{1-6}$-alkoxy, $C_{1-6}$-Alkoxycarbonyl-$C_{1-6}$-alkyloxy-, $C_{2-12}$-Dialkylamino-, 1-Imidazolyl-, Trifluormethyl- oder Cyangruppe sein können,

oder $R_{4a}$l einen Naphthylrest bedeutet

oder $R_{4a}$l einen gesättigten oder ungesättigten heterocyclischen Fünfring mit 1-4 Heteroatomen oder einen gesättigten oder ungesättigten heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere $C_{1-6}$-Alkyl-, $C_{1-6}$-Alkoxy-, $C_{1-6}$-Alkylmercapto-, Hydroxy-, Nitro-, Amino-, Halogen- oder Cyangruppen substituiert oder mit einem Phenylring zu einem Bicyclus kondensiert sein können und

Z Sauerstoff oder Schwefel bedeutet,

und in der im Fall

b), in dem X die Bedeutung $X_b$ hat,

$X_b$ Sauerstoff bedeutet und

$R_1$ die Bedeutung $R_{1b}$ und $R_2$ die Bedeutung $R_{2b}$ hat und

$R_{1b}$ und $R_{2b}$, die gleich oder verschieden sein können, jeweils für Wasserstoff oder $C_{1-6}$-Alkyl stehen,

$R_3$ Wasserstoff oder $C_{1-6}$-Alkyl bedeutet,

$R_4$ die Bedeutung $R_{4b}$ hat und

$R_{4b}$ die Cyanaminogruppe, die 4-Difluormethoxy-3-pyridylgruppe oder eine $C_{1-2}$-Nitroalkylgruppe ist und

Z Sauerstoff oder Schwefel bedeutet,

und in der im Fall

c), in dem X die Bedeutung $X_c$ hat, $X_c$ eine Bindung bedeutet und

$R_1$ die Bedeutung $R_{1c}$ hat und

$R_{1c}$ Wasserstoff, $C_{1-6}$-Alkyl, $C_{2-6}$-Alkenyl oder $C_{3-7}$-Cycloalkyl ist und

$R_2$ die Bedeutung $R_{2c}$ hat und

$R_{2c}$ Wasserstoff, $C_{1-6}$-Alkyl, $C_{2-6}$-Alkenyl oder $C_{1-6}$-Hydroxyalkyl bedeutet, wobei $R_{1c}$ und $R_{2c}$ gleich oder verschieden sein können, oder

$R_{1c}$ und $R_{2c}$ zusammen mit dem von ihnen eingeschlossenen Kohlenstoffatom $C_{3-7}$-Spiroalkyl bedeuten,

$R_3$ Wasserstoff oder $C_{1-6}$-Alkyl bedeutet,

$R_4$ die Bedeutung $R_{4c}$ hat und

$R_{4c}$ die Cyanaminogruppe, die 4-Difluormethoxy-3-pyridylgruppe oder eine $C_{1-2}$-Nitroalkylgruppe ist und

Z Sauerstoff oder Schwefel bedeutet,
sowie deren Salzen und Säureadditionssalzen, dadurch gekennzeichnet, daß man Verbindungen der allgemeinen Formel VI,

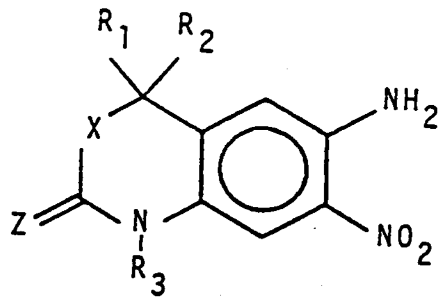

(VI)

in der $R_1$, $R_2$, $R_3$, X und Z die angegebenen Bedeutungen haben, entweder mit Verbindungen der Formel VII

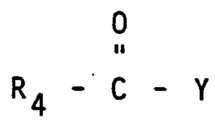

(VII)

in der $R_4$ die angegebene Bedeutung hat und Y Wasserstoff, Hydroxy, Halogen oder eine leicht abspaltbare Gruppe darstellt, umsetzt, und die Reaktionsprodukte anschließend unter sauren Bedingungen hydriert und zyklisiert, wodurch Verbindungen der Formel I erhalten werden, oder mit Raney-Nickel zu Verbindungen der allgemeinen Formel VIII hydriert,

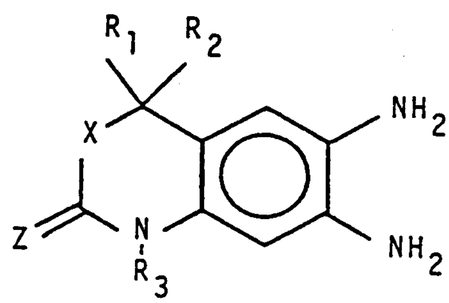

(VIII)

in der $R_1$, $R_2$, $R_3$, X und Z die angegebenen Bedeutungen haben, oder
die Verbindungen der Formel VIII durch Reaktion mit Verbindungen der Formel VII in Verbindungen der Formel IX oder deren Isomere

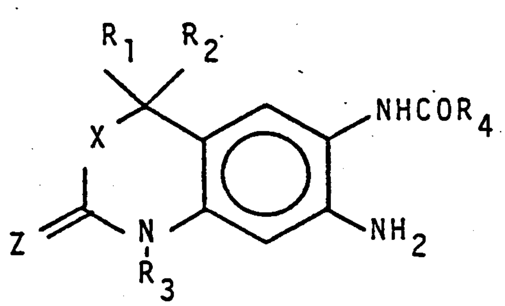

überführt, in der $R_1$, $R_2$, $R_3$, $R_4$, X und Z die angegebenen Bedeutungen haben und durch Zyklisierung in Verbindungen der allgemeinen Formel I umwandelt, und diese gegebenenfalls in Salze oder Säureadditionssalze überführt.

2. Verfahren gemäß Anspruch 1, dadurch gekennzeichnet, daß man 2-Cyanamino-8,8-dimethyl-8-hydro-5H-imidazo(4,5-g)(3,1) benzoxazin-6-on herstellt.

3. Verfahren gemäß Anspruch 1, dadurch gekennzeichnet, daß man 8,8-Dimethyl-2-(4-difluormethoxy-3-pyridyl)-8-hydro-5H-imidazo(4,5-g)(3,1)benzoxazin-6-on herstellt.

4. Verfahren gemäß Anspruch 1, dadurch gekennzeichnet, daß man 2-Cyanamino-7,8-dihydro-3H,5H-imidazo(4,5-g)chinolin-6-on herstellt.

5. Verfahren gemäß Anspruch 1, dadurch gekennzeichnet, daß man 2-((4-Difluormethoxy)-3-pyridyl)-6,7-dihydro-7,7-dimethyl-3H,5H-pyrrolo(2,3-f)-benzimidazol-6-on herstellt.

6. Verfahren gemäß Anspruch 1, dadurch gekennzeichnet, daß man 2-Cyanamino-6,7-dihydro-7,7-dimethyl-3H,5H-pyrrolo(2,3-f)benzimidazol-6-on herstellt.

7. Verwendung einer Verbindung der Formel I gemäß Anspruch 1 oder deren pharmazeutisch verträglichen Salzen oder Säureadditionssalzen zur Herstellung von pharmazeutischen Präparaten und topischen Zubereitungen.

Europäisches Patentamt

# EUROPÄISCHER RECHERCHENBERICHT

Nummer der Anmeldung

**EP 91 11 3388**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y,D | EP-A-0 161 632 (BOEHRINGER MANNHEIM GMBH)<br>* Ansprüche 1, 5-7; Beis& DE-A-3417643 * | 1,7-9 | C 07 D 471/04<br>C 07 D 487/04<br>C 07 D 498/04<br>A 61 K 31/47<br>A 61 K 31/415 //<br>(C 07 D 471/04<br>C 07 D<br>C 07 D 235:00<br>C 07 D 221:00 )<br>(C 07 D 487/04<br>C 07 D<br>C 07 D 235:00<br>C 07 D 209:00 )<br>(C 07 D 498/04<br>C 07 D<br>C 07 D 265:00<br>C 07 D 235:00 ) |
| D,Y | DE-A-3 642 315 (BOEHRINGER MANNHEIM GMBH)<br>* Ansprüche; Beispiel 9 * | 1,7-9 | |
| D,Y | DE-A-3 639 466 (DR. K. THOMAE GMBH)<br>* Ansprüche 1, 5-8, 10; Beispiele 3, 4 * | 1,7-9 | |
| D,Y | DE-A-3 633 861 (DR. K. THOMAE GMBH)<br>* Ansprüche 1, 5-8, 10; Beispiel * | 1,7-9 | |
| D,Y | DE-A-3 701 277 (BOEHRINGER MANNHEIM GMBH)<br>* Ansprüche; Beispiele 1, 4-10 * | 1,7-9 | |
| A,D | DE-A-3 531 678 (BOEHRINGER MANNHEIM GMBH)<br>* Ansprüche * | 1,7-9 | |
| A,D | DE-A-3 524 067 (BOEHRINGER MANNHEIM GMBH)<br>* Ansprüche 1-4 * | 1,7-9 | RECHERCHIERTE SACHGEBIETE (Int. Cl.5) |
| A,D | DE-A-3 501 497 (BOEHRINGER MANNHEIM GMBH)<br>* Ansprüche 1-4 * | 1,7-9 | C 07 D 471/00<br>C 07 D 487/00<br>C 07 D 498/00 |
| A | EP-A-0 186 010 (BOEHRINGER MANNHEIM GMBH)<br>* Ansprüche 1, 6, 8, 9 * | 1,7-9 | |

Der vorliegende Recherchenbericht wurde für alle Patentansprüche erstellt

| Recherchenort | Abschlußdatum der Recherche | Prüfer |
|---|---|---|
| Berlin | 25 November 91 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument